(19) Europäisches Patentamt
European Patent Office
Office européen des brevets

(11) Veröffentlichungsnummer: **0 159 964**
**A2**

# (12) EUROPÄISCHE PATENTANMELDUNG

(21) Anmeldenummer: **85810171.0**

(22) Anmeldetag: **18.04.85**

(51) Int. Cl.⁴: **C 07 D 335/06**, C 07 D 335/04, C 07 D 335/08, A 61 K 31/38

(30) Priorität: **24.04.84 CH 2000/84**

(43) Veröffentlichungstag der Anmeldung: **30.10.85 Patentblatt 85/44**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Ilvespää, Atso, Dr., Spitzwaldstrasse 154, CH-4123 Allschwil (CH)**
Erfinder: **Haas, Georges, Dr., Im Rehwechsel 24, CH-4102 Binningen (CH)**

(54) **3-Substituierte Thiopyrone.**

(57) Die Erfindung betrifft neue Thiopyrone, insbesondere neue Benzothiopyranone der allgemeinen Formel

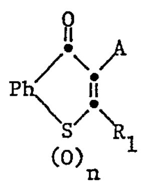

(I)

worin $R_1$ Wasserstoff oder einen aliphatischen Rest bedeutet, n für 0, 1 oder 2 steht, Ph substituiertes oder unsubstituiertes 1,2-Phenylen darstellt und A eine Gruppe der Formel $-NH-CO-R$ bedeutet, in der R Carboxy oder verestertes Carboxy darstellt, und ihre Salze, Verfahren zur Herstellung von Verbindungen der Formel (I) und ihrer Salze, solche Verbindungen enthaltende pharmazeutische Präparate sowie die Verwendung von Verbindungen der Formel (I) und ihrer Salze als Arzneimittelwirkstoffe und/oder zur Herstellung pharmazeutischer Präparate. Die Verbindungen der Formel (I) und ihre Salze haben antiallergische Eigenschaften.

EP 0 159 964 A2

 3159964

CIBA-GEIGY AG
Basel (Schweiz)

4-14842/+

3-Substituierte Thiopyrone

Die Erfindung betrifft neue Benzothiopyranone der allgemeinen Formel

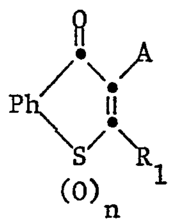

(I),

worin $R_1$ Wasserstoff oder einen aliphatischen Rest bedeutet, n für 0, 1 oder 2 steht, Ph substituiertes oder unsubstituiertes 1,2-Phenylen darstellt und A eine Gruppe der Formel -NH-CO-R bedeutet, in der R Carboxy oder verestertes Carboxy darstellt, und ihre Salze, Verfahren zur Herstellung von Verbindungen der Formel (I) und ihrer Salze, solche Verbindungen enthaltende pharmazeutische Präparate sowie die Verwendung von Verbindungen der Formel (I) und ihrer Salze als Arzneimittelwirkstoffe und/oder zur Herstellung pharmazeutischer Präparate.

Ein aliphatischer Rest $R_1$ ist insbesondere unsubstituiert und gesättigt, ferner ungesättigt, und stellt in erster Linie einen Niederalkylrest, ferner Niederalkenyl dar.

1,2-Phenylen kann ein- oder mehrfach, wie zweifach, z.B. durch einen aliphatischen Rest, Hydroxy, mit einem aliphatischen Alkohol verethertes Hydroxy, durch einen aliphatischen Rest substituiertes Mercapto bzw. Sulfeno bzw. Sulfino, Acyl, Nitro und/oder Halogen, substituiert sein.

Aliphatische Reste als Substituenten von Ph sind z.B. Niederalkyl, Niederalkenyl, Niederalkinyl, zwei benachbarte C-Atome überbrückendes 3- oder 4-gliedriges Niederalkylen, Hydroxyniederalkyl oder Halogenniederalkyl.

Mit einem aliphatischen Alkohol verethertes Hydroxy als Substituent von Ph ist in erster Linie Niederalkoxy, Hydroxyniederalkoxy oder Niederalkenyloxy, während mit einem aliphatischen Rest substituiertes Mercapto bzw. Sulfeno bzw. Sulfino in erster Linie Niederalkylthio, Niederalkansulfinyl bzw. Niederalkansulfonyl darstellt.

Acyl leitet sich insbesondere von aliphatischen Carbonsäuren ab und bedeutet in erster Linie Niederalkanoyl oder Niederalkanoyloxy.

Verestertes Carboxy ist beispielsweise mit einem aliphatischen oder aromatischen, insbesondere monocyclischen, Alkohol verestertes Carboxy, in erster Linie Niederalkoxycarbonyl oder unsubstituiertes bzw. ein- oder mehrfach, z.B. durch wie für Ph angegebene Substituenten, substituiertes Phenoxycarbonyl.

Vor- und nachstehend sind unter mit "nieder" bezeichneten organischen Resten, Gruppen und Verbindungen solche zu verstehen, die bis und mit 7, vor allem bis und mit 4 Kohlenstoffatome enthalten.

Die im Rahmen des vorliegenden Textes verwendeten Allgemeindefinitionen haben in erster Linie die folgenden Bedeutungen:

Niederalkyl ist z.B. Methyl, Ethyl, n-Propyl, Isopropyl, n-Butyl, Isobutyl, sek-Butyl oder tert-Butyl und umfasst ferner entsprechende Pentyl-, Hexyl- oder Heptylreste.

Niederalkenyl weist z.B. mindestens 3 C-Atome und die Doppelbindung insbesondere in höher als in der α-Stellung auf und ist z.B. 2-Propenyl, während Niederalkinyl z.B. Propargyl bedeutet.

3-oder 4-gliedriges Niederalkylen weist mindestens 3 C-Atome auf, ist in erster Linie geradkettig, ferner verzweigt, und ist z.B. Tri- oder Tetramethylen.

Hydroxyniederalkyl ist z.B. Hydroxymethyl oder 2-Hydroxyethyl.

Halogenniederalkyl ist z.B. Chlormethyl, Trifluormethyl oder 1,1,2-Trifluor-2-chlorethyl.

Niederalkoxy ist z.B. Methoxy, Ethoxy, n-Propyloxy, Isopropyloxy, n-Butyloxy, Isobutyloxy, sek-Butyloxy oder tert-Butyloxy, ferner entsprechende Pentyloxy-, Hexyloxy- oder Heptyloxyreste.

Hydroxyniederalkoxy kann bis zu 3 Hydroxygruppen aufweisen, die vorzugsweise in höherer als der α-Stellung gebunden sind, und ist z.B. 2-Hydroxyethoxy oder 3-Hydroxypropoxy.

Niederalkenyloxy weist die Doppelbindung insbesondere in höherer als der α-Stellung auf und ist z.B. Allyloxy, But—2-en- oder But-3-enyloxy.

Niederalkylthio ist z.B. Methyl-, Ethyl-, n-Propyl-, Isopropyl-, n-Butyl-, Isobutyl-, sek.-Butyl- oder tert.-Butylthio. Niederalkansulfinyl bzw. -sulfonyl ist z.B. Methan-, Ethan-, n-Propan- oder Isopropansulfinyl bzw. -sulfonyl.

Niederalkanoyl ist z.B. Formyl, Acetyl, Propionyl, Butyryl, Isobutyryl oder Pivaloyl.

Niederalkanoyloxy ist z.B. Acetyloxy, Propionyloxy, Butyryloxy, Isobutyryloxy oder Pivaloyloxy.

Halogen ist z.B. Halogen der Atomnummer bis und mit 35, wie Fluor, Chlor oder Brom, und umfasst ferner Iod.

Salze von erfindungsgemässen Verbindungen der Formel (I) sind vorzugsweise pharmazeutisch verwendbare Salze. Bedeutet R beispielsweise Carboxy, können entsprechende Salze mit Basen gebildet werden. Derartige Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclische Amine, wie Niederalkylamine, z.B. Mono-, Di- bzw. Triniederalkylamine, wie Hydroxyniederalkylamine, z.B. Mono-, Di- bzw. Trihydroxyniederalkylamine, wie Hydroxyniederalkyl-niederalkylamine oder wie Polyhydroxyniederalkylamine. Cyclische Amine sind z.B. Morpholin, Thiomorpholin, Piperidin oder Pyrrolidin. Als Mononiederalkylamine kommen beispielsweise Ethyl- oder tert-Butylamin, als Diniederalkylamine beispielsweise Diethyl- oder Diisopropylamin, als Triniederalkylamine beispielsweise Trimethyl- oder Triethylamin in Betracht. Entsprechende Hydroxyniederalkylamine sind z.B. Mono-, Di- bzw. Triethanolamin oder Tris-(hydroxymethyl)-methylamin, und Hydroxyniederalkyl-niederalkyl-amine sind z.B. N,N-Dimethylamino- oder N,N-Diethylamino-ethanol, ferner Glucosamin oder N-Methyl-D-glucamin als Polyhydroxyniederalkylamine. Umfasst sind ferner für pharmazeutische Verwendungen ungeeignete Salze, da diese für die Isolierung bzw. Reinigung freier erfindungsgemässer Verbindungen sowie deren pharmazeutischer verwendbarer Salze verwendet werden können.

Die Verbindungen der Formel (I) weisen wertvolle pharmakologische Eigenschaften auf. Insbesondere besitzen sie eine ausgeprägte antiallergische Aktivität. Diese lässt sich z.B. im passiven kutanen Anaphylaxie-Test (PCA-Test) an der Ratte im Dosisbereich von etwa 0,1 bis etwa 100 mg/kg bei oraler Applikation nachweisen, wobei nach der Methodik von G.E. Davies u. D.P. Evans, Int. Arch. Allergy 45, 467 (1973) verfahren wird. Die PCA-Reaktion wird analog Z. Ovary, Progr. Allergy 5, 459 (1958) induziert.

Infolgedessen können die Wirkstoffe der Formel (I) z.B. als Antiallergika in Arzneimitteln zur prophylaktischen und therapeutischen Behandlung verschiedenartiger allergischer Erkrankungen, insbesondere von Asthma bronchiale und allergischer Rhinitis, verwendet werden.

Ein weiterer Gegenstand der Erfindung sind somit die Verbindungen der Formel I und deren pharmazeutisch verwendbaren Salze zur Anwendung in einem Verfahren zur therapeutischen und prophylaktischen Behandlung des menschlichen Körpers sowie die Verwendung der Verbindungen zur Herstellung von Arzneimitteln.

Die Erfindung betrifft insbesondere Verbindungen der Formel (I), worin $R_1$ Wasserstoff, Niederalkyl oder Niederalkenyl bedeutet, n für 0, 1 oder 2 steht, Ph ein- oder mehrfach durch Niederalkyl, Niederalkenyl, Niederalkinyl, zwei benachbarte C-Atome überbrückendes 3- oder 4-gliedriges Niederalkylen, Hydroxyniederalkyl, Halogenniederalkyl, Hydroxy, Niederalkoxy, Hydroxyniederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes oder unsubstituiertes 1,2-Phenylen darstellt und A eine Gruppe der Formel -NH-CO-R bedeutet, in der R Carboxy, Niederalkoxycarbonyl, unsubstituiertes oder ein- oder mehrfach durch Niederalkyl, Niederalkenyl, Niederalkinyl, zwei benachbarte C-Atome überbrückendes 3- oder 4-gliedriges Niederalkylen, Hydroxyniederalkyl, Halogenniederalkyl, Hydroxy, Niederalkoxy, Hydroxyniederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes Phenoxycarbonyl darstellt, und Salze, insbesondere pharmazeutisch verwendbare Salze.

Die Erfindung betrifft insbesondere Verbindungen der Formel (I), worin $R_1$ Wasserstoff oder Niederalkyl bedeutet, n für 0, 1 oder 2 steht, Ph ein- oder mehrfach durch Niederalkyl, Niederalkenyl, zwei benachbarte C-Atome überbrückendes 3- oder 4-gliedriges Niederalkylen, Hydroxyniederalkyl, Hydroxy, Niederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes oder unsubstituiertes 1,2-Phenylen darstellt und A eine Gruppe der Formel -NH-CO-R bedeutet, in der R Carboxy oder Niederalkoxycarbonyl darstellt, und Salze, insbesondere pharmazeutische verwendbare Salze.

Die Erfindung betrifft insbesondere Verbindungen der Formel (I), worin $R_1$ Wasserstoff oder Niederalkyl bedeutet, n für 0 steht, Ph ein- oder mehrfach durch Niederalkyl, Niederalkenyl, zwei benachbarte C-Atome überbrückendes 3- oder 4-gliedriges Niederalkylen, Niederalkoxy und/oder Halogen substituiertes oder unsubstituiertes 1,2-Phenylen darstellt und A eine Gruppe der Formel -NH-CO-R bedeutet, in der R Carboxy oder Niederalkoxycarbonyl darstellt, und Salze, insbesondere pharmazeutisch verwendbare Salze.

Die Erfindung betrifft vor allem Verbindungen der Formel (I), worin $R_1$ Wasserstoff bedeutet, n für 0 steht, Ph ein- oder mehrfach, wie zweifach, durch Niederalkyl, insbesondere mit bis und mit 7 C-Atomen, wie Methyl, zwei benachbarte C-Atome überbrückendes 3- oder 4-gliedriges Niederalkylen mit 3 bis und mit 7 C-Atomen, wie Tri- oder Tetramethylen, Niederalkoxy, z.B. mit bis und mit 4 C-Atomen, wie Methoxy, und/oder Halogen, insbesondere bis und mit Atomnummer 35, wie Chlor, substituiertes oder unsubstituiertes 1,2-Phenylen darstellt und A eine Gruppe der Formel -NH-CO-R bedeutet, in der R Carboxy oder Niederalkoxycarbonyl, insbesondere mit 2 bis und mit 5 C-Atomen, wie Methoxycarbonyl, darstellt, und Salze, insbesondere pharmazeutisch verwendbare Salze.

Die Erfindung betrifft bevorzugt Verbindungen der Formel

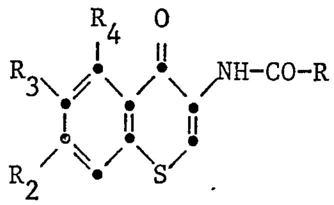

(Ia),

worin R Carboxy bedeutet, $R_2$ und $R_4$ Wasserstoff darstellen und $R_3$ Wasserstoff, Niederalkyl mit bis und mit 4 C-Atomen, wie Methyl, oder Niederalkoxy mit bis und mit 4 C-Atomen, wie Methoxy, bedeutet oder worin R Carboxy darstellt, $R_2$ und $R_3$ gemeinsam Tri- oder Tetramethylen darstellen und $R_4$ Wasserstoff bedeutet, und Salze, insbesondere pharmazeutisch verwendbare Salze.

Die Erfindung betrifft in erster Linie Verbindungen der Formel (Ia), worin R Carboxy bedeutet, $R_2$ und $R_4$ Wasserstoff bedeuten und $R_3$ Niederalkyl mit bis und mit 4 C-Atomen bedeutet oder worin $R_2$ und $R_3$ gemeinsam Tri- oder Tetramethylen bedeuten und $R_4$ Wasserstoff ist oder worin $R_3$ und $R_4$ gemeinsam Tri- oder Tetramethylen bedeuten und $R_2$ Wasserstoff ist, und ihre Salze, insbesondere pharmazeutisch verwendbare Salze.

Die Erfindung betrifft in erster Linie Verbindungen der Formel (Ia), worin R Carboxy bedeutet, $R_2$ und $R_4$ Wasserstoff bedeuten und $R_3$ Niederalkyl mit bis und mit 4 C-Atomen, insbesondere Methyl, bedeutet oder worin $R_2$ und $R_3$ gemeinsam Tri- oder Tetramethylen bedeuten und $R_4$ Wasserstoff ist, und ihre Salze, insbesondere pharmazeutisch verwendbare Salze.

Die Erfindung betrifft namentlich die in den Beispielen genannten neuen Verbindungen und ihre Salze, vor allem pharmazeutisch verwendbare Salze, sowie die in den Beispielen beschriebenen Verfahren zu ihrer Herstellung.

Die neuen Verbindungen können nach an sich bekannter Weise hergestellt werden.

Eine Arbeitsweise besteht beispielsweise darin, dass man in einer Verbindung der Formel

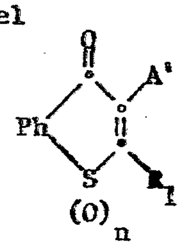

(II),

worin n, Ph und $R_1$ die angegebenen Bedeutungen haben und A' eine in A überführbare Gruppe bedeutet, A' in A überführt und, wenn erwünscht, eine verfahrensgemäss erhältliche freie Verbindung in eine andere freie Verbindung überführt, ein verfahrensgemäss erhältliches Salz in die freie Verbindung oder in ein anderes Salz überführt, eine verfahrensgemäss erhältliche freie Verbindung in ein Salz überführt und/oder gewünschtenfalls ein verfahrensgemäss erhältliches Isomerengemisch in seine Komponenten auftrennt.

Eine in A überführbare Gruppe A' bedeutet beispielsweise die Aminogruppe.

Eine bevorzugte Ausführungsform des Verfahrens besteht beispielsweise darin, dass man eine Verbindung der Formel

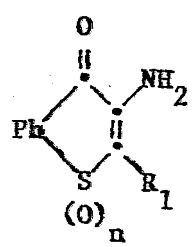

(IIa),

worin n, Ph und $R_1$ die angegebenen Bedeutungen haben, oder ein Salz davon mit einer Verbindung der Formel $R-X_1$ (IIb), worin R die angegebene Bedeutung hat und $X_1$ Carboxy oder funktionell abgewandeltes Carboxy bedeutet, umsetzt.

Salze von Verbindungen der Formel (IIa) sind beispielsweise Säureadditionssalze, wie entsprechende Hydrohalogenide oder Niederalkanoate. Unter funktionell abgewandeltem Carboxy ist z.B. reaktionsfähiges verestertes, anhydridisiertes oder ferner amidiertes Carboxy zu verstehen. Dabei kann verestertes Carboxy $X_1$ beispielsweise die eingangs für R angegebenen Bedeutungen haben, während als anhydrisiertes Carboxy beispielsweise mit einer Mineralsäure, wie Halogenwasserstoffsäure, oder mit einer Carbonsäure, wie Niederalkancarbonsäure oder Kohlensäurehalogenidniederalkylhalbester, anhydridisiertes Carboxy in Betracht kommt.

Amidiertes Carboxy weist beispielsweise eine freie, mono- oder disubstituierte Aminogruppe auf. Eine monosubstituierte Aminogruppe ist z.B. durch einen aliphatischen Rest, durch einen araliphatischen Rest oder durch einen aromatischen Rest monosubstituiert. Eine disubstituierte Aminogruppe weist als Substituenten aliphatische und/oder araliphatische Reste auf.

Ein aliphatischer Rest als Substituent von Carbamoyl ist insbesondere ein gesättigter aliphatischer Rest, in erster Linie Niederalkyl. Ein entsprechender araliphatischer Rest weist als Arylteil insbesondere monocyclisches Aryl, wie gegebenenfalls substituiertes Phenyl, auf und der aliphatische Teil ist gesättigt und bedeutet in erster Linie Niederalkyl. Entsprechende Substituenten sind in erster Linie im Phenylteil substituiertes oder unsubstituiertes Phenylniederalkyl. Ein aromatischer Rest als Substituent von Carbamoyl ist insbesondere ein monocyclischer aromatischer Rest und bedeutet in erster Linie wie vorstehend für Phenyl angegebenes substituiertes oder unsubstituiertes Phenyl. Substituiertes Phenyl aufweisendes Carbamoyl kann im Phenylteil jeweil ein- oder mehrfach durch Niederalkyl, Niederalkoxy, Hydroxy, Niederalkanoyloxy, Nitro, Halogen und/oder Trifluormethyl substituiert sein. Als aliphatischer Rest in N,N-disubstituiertem Carbamoyl kommt ebenfalls ein Alkylen oder durch Monoaza, N-alkyliertes Monoaza, Monooxa bzw. Monothia unterbrochenes Alkylen, in erster

Linie entsprechendes Niederalkylen, in Betracht. Als Beispiele für derartig amidiertes Carboxy sind z.B. Carbamoyl, N-Mono- oder N,N-Diniederalkyl-carbamoyl, N-Mono- oder N,N-Di-niederalkylphenyl-carbamoyl, N-Mono-phenyl-carbamoyl, N-(5-Tetrazolyl)-carbamoyl, Niederalkylencarbamoyl oder durch Monoaza, N'-Niederalkylaza, Monooxa bzw. Monothia unterbrochenes Niederalkylencarbamoyl zu nennen.

Für die vorstehende Umsetzung werden insbesondere solche Verbindungen der Formel (IIb) verwendet, worin $X_1$ Carboxy oder Halogencarbonyl, wie Chlorcarbonyl, bedeutet.

Die Amidierung kann in der für analoge Reaktionen bekannten Weise durchgeführt werden. Dabei kann beispielsweise ein Kondensationsmittel erforderlich sein. Die verfahrensgemässe Umsetzung (N-Acylierung) wird erforderlichenfalls in Gegenwart eines, insbesondere basischen, Kondensationsmittels durchgeführt. Als Basen kommen beispielsweise Alkalimetallhydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide sowie carbocyclische Amine in Frage. Beispielhaft seien Natrium-hydroxid, -hydrid, -amid, Kalium-tert-butylat, -carbonat, Lithium-triphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyl-trimethylammoniumhydroxid, 1,5-Diazabicyclo[4.3.0]non-5-en (DBN) sowie 1,8-Diaza-bicyclo[5.4.0]-undec-7-en (DBU) genannt. Den Kondensationsmitteln zuzurechnen sind die bei der Bildung von Amidbindungen üblichen Dehydratisierungsmittel, die insbesondere dann verwendet werden, wenn $X_1$ der Formel IIb Carboxy bedeutet. Dabei können beispielsweise in situ reaktionsfähige Carboxyderivate der Formel IIb, insbesondere entsprechende aktivierte Ester bzw. Amide gebildet werden. Geeignete Dehydratisierungsmittel sind beispielsweise Carbodiimide, z.B. N,N'-Diniederalkyl- oder N,N'-Dicycloalkyl-carbodimid, wie N,N'-Diethyl-, N,N'-Diisopropyl- oder N,N'-Dicyclohexyl-carbodiimide, vorteilhaft unter Zusatz von N-Hydroxysuccinimid oder

gegebenenfalls, z.B. durch Halogen, Niederalkyl oder Niederalkoxy, substituiertes 1-Hydroxy-benzotriazol oder N-Hydroxy-5-norbonen-2,3-dicarboxamid, N,N'-Diimidazolcarbonyl, eine geeignete Phosphoryl- bzw. Phosphinverbindung z.B. Diethylphosphonylcyanid, Diphenylphosphonylazid oder Triphenylphosphin-disulfid, ein 1-Niederalkyl-2-halogen-pyridinium-halogenid, z.B. 1-Methyl-2-chlor-pyridinium-iodid, ein geeignetes 1,2-Dihydrochinolin, z.B. N-Ethoxycarbonyl-2-ethoxy-1,2-dihydrochinolin, oder 1,1'-(Carbonyldioxy)-dibenzotriazol. Bei der Reaktion mit einer Verbindung der Formel (IIb), worin R eine veresterte Carboxygruppe bedeutet und $X_1$ Halogencarbonyl darstellt, wird beispielsweise ein basisches Kondensationsmittel, wie eine organische Stickstoffbase, z.B. Triethylamin oder Pyridin, oder wie ein Alkalimetallhydroxid oder -carbonat, z.B. Natrium- oder Kaliumhydroxid, verwendet. Bei der Umsetzung mit einer entsprechenden Verbindung der Formel (IIb), worin $X_1$ Carboxy bedeutet, kann z.B. auch ein Carbodiimid oder Isonitril, wie Dicyclohexylcarbodiimid oder tert-Butylisonitril, als Dehydratisierungsmittel, ferner eine Mineralsäure, wie Chlorwasserstoffsäure, oder ein Säureanhydrid, wie Phosphorpentoxid, eingesetzt werden. Die Amidierung wird z.B. in An- oder Abwesenheit eines Lösungs- oder Verdünnungsmittels, wie eines halogenierten Kohlenwasserstoffs, z.B. Chloroform, durchgeführt. Bevorzugt werden äquimolare Mengen von entsprechenden Verbindungen der Formeln (IIa) und (IIb) eingesetzt.

Die Ausgangsstoffe der Formeln (IIa) und (IIb) sind bekannt oder können analog üblichen Verfahren hergestellt werden.

So gelangt man beispielsweise zu Verbindungen der Formel (IIa), indem Verbindungen der Formel

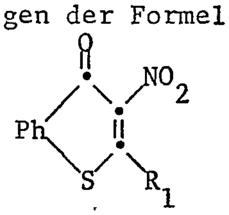

(IIf)

zu Verbindungen der Formel (IIa), worin n null bedeutet, reduziert werden. Die Reduktion wird beispielsweise mit Wasserstoff unter der katalytischen Wirkung von Palladium/Kohle durchgeführt.

Zur Herstellung von Verbindungen der Formel (IIa), worin n 1 oder 2 darstellt, wird z.B. von Verbindungen der Formel (IIa), worin n null ist, ausgegangen und die Aminogruppe geschützt, beispielsweise durch Acetylierung mit Acetanhydrid. Bei der Verwendung von etwa einem Aequivalent eines Oxidationsmittels, z.B. von m-Chlorperbenzoesäure, können Verbindungen der Formel (IIa) erhalten werden, wobei n 1 bedeutet, während die Oxidation mit mindestens 2 Mol an Oxidationsmittel zu entsprechenden Verbindungen der Formel (IIa) führt, worin n 2 bedeutet.

Im nächsten Reaktionsschritt wird die Aminoschutzgruppe wieder abgespalten. So wird beispielsweise eine Acetylgruppe durch Behandeln mit ethanolischer Salzsäure hydrolytisch abgespalten.

Eine bevorzugte Ausführungsform zur Herstellung von Verbindungen der Formel (IIa), worin $R_1$ für Wasserstoff steht, besteht darin, dass man Verbindungen der Formel

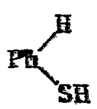

(IId)

oder deren Salze mit Halogen-, wie Brompropionsäure umsetzt und die so erhältlichen Verbindungen der Formel

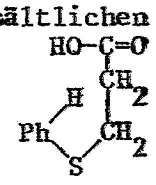

(IIe)

in Gegenwart einer starken Säure, wie Schwefelsäure, zu Verbindungen der Formel

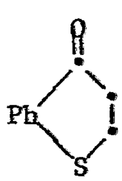

(IIf)

cyclisiert. Diese werden anschliessend mit z.B. Isopentylnitrit in Kaliumhydroxidlösung behandelt und die so erhältlichen Verbindungen der Formel

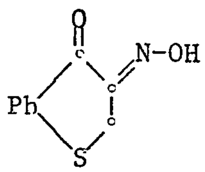

(IIg)

mit Chlor- oder Bromwasserstoffsäure umgesetzt. Dabei fallen entsprechende Verbindungen der Formel

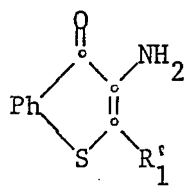

(IIh)

an, worin $R_1'$ Wasserstoff oder Chlor bzw. Brom bedeutet. Durch Reduktion z.B. mit Wasserstoff in Gegenwart von Palladium /Kohle, kann das Chlor- bzw. Bromatom in entsprechenden Verbindungen der Formel (IIh) durch Wasserstoff ersetzt werden.

Aus den so erhältlichen Verbindungen der Formel (IIa), worin n null und $R_1$ Wasserstoff bedeuten, kann man in der vorstehend beschriebenen Weise zu entsprechenden S-oxidierten Verbindungen gelangen.

Eine weitere in A überführbare Gruppe A' bedeutet beispielsweise eine Gruppe der Formel $-NH-CO-X_2$, wobei $X_2$ einen durch Solvolyse, wie Hydrolyse oder Alkoholyse, in R überführbaren Rest darstellt. Als entsprechender Rest $X_2$ kommt z.B. von R verschiedenes funktionell abgewandeltes Carboxy in Frage; zu nennen sind in erster Linie Cyano, anhydridisiertes Carboxy, wie Halogencarbonyl, Niederalkanoyloxycarbonyl oder Niederalkoxycarbonyloxycarbonyl, wie vorstehend aufgeführtes amidiertes Carboxy, gegebenenfalls substituiertes Amidino, wie Niederalkylamidino, gegebenenfalls verestertes Thiocarboxy, wie Niederalkylthio-carbonyl, gegebenenfalls verestertes Dithiocarboxy, gegebenenfalls substituiertes Thiocarbamoyl, wie Mono- oder Diniederalkyl-thiocarbamoyl, gegebenenfalls verestertes oder anhydridisiertes Carboximidoyl, wie Niederalkoxy- oder Halogeniminocarbonyl, oder sich von Orthoameisensäureester ableitende Reste, wie Triniederalkoxy- oder Trihalogenmethyl.

Solvolysemittel sind beispielsweise Wasser oder der gewünschten veresterten Carboxygruppe entsprechende Alkohole.

Die Behandlung mit einem entsprechenden Solvolysemittel wird gegebenenfalls in Gegenwart einer Säure oder Base, gegebenenfalls unter Kühlen oder Erwärmen und erforderlichenfalls in einem inerten Lösungs- oder Verdünnungsmittel durchgeführt. Als Säuren eignen sich beispielsweise anorganische oder organische Protonsäuren, wie Mineralsäuren, z.B. Schwefelsäure oder Halogenwasserstoffsäure, wie Sulfonsäuren, z.B. Niederalkan- oder gegebenenfalls substituierte Benzolsulfonsäure, oder wie Carbonsäuren, wie Niederalkancarbonsäuren. Als Basen können beispielsweise Hydroxide, wie Alkalimetallhydroxide, verwendet werden.

So können beispielsweise die Cyanogruppe, wie vorstehend aufgeführtes amidiertes Carboxy, gegebenenfalls verestertes Thiocarboxy, gegebenenfalls verestertes Dithiocarboxy, gegebenenfalls substituiertes Thiocarbamoyl, gegebenenfalls verestertes oder anhydridisiertes Carboximidoyl, oder sich von Orthoameisensäure ableitende Reste $X_2$, gegebenenfalls in Gegenwart einer Protonsäure zu Carboxy R hydrolysiert werden, während beispielsweise Cyano, gegebenenfalls S-verestertes Thiocarboxy oder anhydridisiertes Carboxy $X_2$ mit einem geeigneten Alkohol, gegebenenfalls in Gegenwart einer Protonsäure, zu verestertem Carboxy R alkoholysiert werden können.

Die Abspaltung entsprechender Aminoschutzgruppen erfolgt in erster Linie acidolytisch oder reduktiv. Als Säuren kommen beispielsweise gegebenenfalls substituierte Niederalkancarbonsäuren, wie Trifluoressigsäure, Halogenwasserstoffsäuren, wie Fluorwasserstoffsäure, oder Gemische aus Halogenwasserstoffsäure mit Niederalkancarbonsäuren, wie Bromwasserstoff- und Essigsäure-Gemische, in Frage. Die reduktive Abspaltung erfolgt z.B. durch Reduktion mit in statu nascendi erzeugtem Wasserstoff oder durch katalytisch aktivierten Wasserstoff. Beispielsweise wird Wasserstoff in Systemen, wie Zink-Essigsäure oder

Natrium-Ammoniak, erzeugt, während als Katalysator für die Aktivierung von Wasserstoff z.B. ein Edelmetall oder ein Derivat davon, wie Platin, Rhodium, Palladium oder Platinoxid, ferner Raney-Nickel, verwendet wird.

Das Abspalten der Aminoschutzgruppe erfolgt in An- oder Abwesenheit eines inerten Lösungs- oder Verdünnungsmittels, erforderlichenfalls unter Kühlen und/oder Erwärmen.

Ferner kann A' oxidativ in A überführt werden, wobei A' eine Gruppe der Formel $-NH-CO-X_2$ und $X_2$ gegebenenfalls verestertes oder verethertes Hydroxymethyl oder gegebenenfalls hydratisiertes Formyl darstellen.

Verestertes Hydroxymethyl ist beispielsweise mit einer aliphatischen Carbonsäure verestertes Hydroxymethyl. Als aliphatische Carbonsäuren kommen insbesondere Niederalkancarbonsäuren in Frage.

Verethertes Hydroxymethyl ist beispielsweise mit einem aliphatischen Alkohol, der in erster Linie gesättigt ist, insbesondere mit einem Niederalkanol, verethertes Hydroxymethyl.

Gegebenenfalls hydratisiertes Formyl kann vorteilhaft im Verlauf der Oxidationsreaktion, z.B. aus einer gegebenenfalls veresterten Hydroxymethylgruppe, in situ gebildet oder aus einem seiner funktionellen Derivate, z.B. einem seiner Acetale oder Imine, in Freiheit gesetzt werden. Entsprechende veresterte Hydroxymethylgruppen sind beispielsweise an der Hydroxygruppe mit einer Mineralsäure, wie Halogenwasserstoffsäure, z.B. mit Chlor- oder Bromwasserstoffsäure, oder mit einer Carbonsäure, wie Niederalkancarbonsäure oder der gegebenenfalls substituierten Benzoesäure, veresterte Hydroxymethylgruppen. Acetalisierte Formylgruppen sind beispielsweise mit Niederalkanolen oder einem Niederalkandiol acetalisierte Formylgruppen, wie Dimethoxy-, Diethoxy-, Ethylendioxy- oder Trimethylendioxygruppen. Iminomethylgruppen sind beispielsweise gegebenenfalls substituierte N-Benzylimino- oder

N-(2-Benzothiazolyl)-imino-methyl. Hydroxymethyl- oder gegebenenfalls hydratisierte Formylgruppen $X_2$ können zu Carboxy R und veretherte Hydroxymethylgruppen zu verestertem Carboxy R oxidiert werden.

Die Oxidation kann in üblicher Weise durch Umsetzung mit einem geeigneten Oxidationsmittel erfolgen. Geeignete Oxidationsmittel sind insbesondere oxidierende Schwermetallverbindungen, wie Silberverbindungen, z.B. Silbernitrat oder Silberpicolinat, Sauerstoffsäuren von Schwermetallen, z.B. von Mangan-IV, Mangan-VII, Chrom-VI und Eisen-III, oder von Halogenen bzw. deren Anhydride oder Salze, wie Chromsäure, Chromdioxid, Kaliumdichromat, Kaliumpermanganat, Mangandioxid, Kaliumferrat, Natriumchlorit in Gegenwart von Sulfaminsäure, Natriumhypochlorit in Gegenwart von Nickelchlorid oder Natriumiodat, Natriumperiodat oder Bleitetraacetat. Die Umsetzung mit diesen Oxidationsmitteln erfolgt in üblicher Weise, beispielsweise in einem inerten Lösungsmittel, wie Aceton, Essigsäure, Pyridin oder Wasser, oder einem, vorzugsweise wässrigen, inerten Lösungsmittelgemisch, bei Normaltemperatur oder erforderlichenfalls unter Kühlen oder Erwärmen, z.B. bei etwa 0°C bis etwa 100°C. Die Oxidation von gegebenenfalls veretherten Hydroxymethylgruppen $X_2$ zu gegebenenfalls veresterten Carboxygruppen R wird z.B. vorteilhaft mit Kaliumpermanganat in wässrigem Pyridin oder Aceton bei Raumtemperatur vorgenommen. Acetalisierte Formylgruppen $X_2$ und Iminomethylgruppen $X_2$ werden vorzugsweise sauer oxidiert, z.B. mit Kaliumdichromat in Schwefelsäure, während man für die Oxidation der Formylgruppe vorzugsweise Kaliumferrat in alkalischem Milieu, z.B. bei pH = 10-13, z.B. 11,5, oder organische Silbersalze, wie Silberpicolinat, verwendet.

Die Ausgangsstoffe der Formel (II) können, sofern sie neu sind, nach an sich bekannten Methoden hergestellt werden.

So setzt man beispielsweise Verbindungen der Formel

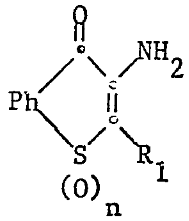

(IIIa)

mit einem Aequivalent einer Verbindung der Formel $X_1$-$X_2$ (IIIb), worin $X_1$ und $X_2$ die vorstehend angegebenen Bedeutungen haben, um.

Für die vorstehende Umsetzung werden insbesondere solche Verbindungen der Formel (IIIb) verwendet, worin $X_1$ Carboxy oder Halogencarbonyl bedeutet.

Die neuen Verbindungen können ferner hergestellt werden, indem man eine Verbindung der Formel

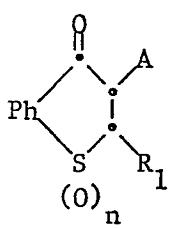

(IV),

worin n, Ph, A und $R_1$ die angegebenen Bedeutungen haben, oder ein Salz davon zu der entsprechenden Verbindung der Formel (I) dehydriert und, wenn erwünscht, eine verfahrensgemäss erhältliche freie Verbindung in eine andere freie Verbindung überführt, ein verfahrensgemäss erhältliches Salz in die freie Verbindung oder in ein anderes Salz überführt, eine verfahrensgemäss erhältliche freie Verbindung in ein Salz überführt und/oder gewünschtenfalls ein verfahrensgemäss erhältliches Isomerengemisch in seine Komponenten auftrennt.

Die Dehydrierung von Verbindungen der Formel (IV) oder deren Salzen erfolgt in an sich bekannter Weise, vorteilhaft bei erhöhter Temperatur, beispielsweise in einem Temperaturintervall von etwa 50° bis etwa 300°C, mit Hilfe eines Dehydrierungsmittels. Solche Mittel sind beispielsweise Dehydrierungskatalysatoren, z.B. Nebengruppenelemente, vorzugsweise solche der VIII. Nebengruppe, wie Palladium oder Platin, oder entsprechende Salze, wie Ruthenium-triphenyl-phosphid-chlorid, wobei die Katalysatoren auf geeigneten Trägermaterialien, wie Aktivkohle, Aluminiumoxid oder Siliciumdioxid, aufgezogen sein können. Weitere geeignete Dehydrierungsmittel sind beispielsweise Chinone, wie p-Benzochinone, z.B. Tetrachlor-p-benzochinon oder 2,3-Dichlor-5,6-dicyano-p-benzochinon, oder wie Anthrachinone, z.B. Phenanthren-9,10-chinon, N-Halogensuccinimide, wie N-Chlorsuccinimid, oder Selenderivate, wie Selendioxid oder Diphenylselenium-bis-trifluoracetat, ferner Selen oder Schwefel. Als Dehydrierungsmittel kann man vor allem auch Trityliumsalze, wie Tritylimperchlorat oder -tetrafluoroborat, verwenden.

Die Umsetzung wird gegebenenfalls in einem inerten hochsiedenden Lösungsmittel, wie in einem Ether, z.B. Diphenylether, erforderlichenfalls bei erhöhter Temperatur unter Druck, in einem geschlossenen Gefäss und/oder unter Inertgas, z.B. Stickstoff, durchgeführt.

Die Ausgangsverbindungen der Formel (IV) oder deren Salze können nach an sich bekannten Methoden hergestellt werden. So geht man beispielsweise von Verbindungen der Formel

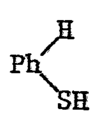

(IVa)

aus und setzt diese mit Verbindungen der Formel $R_1-\overset{Hal}{CH}-CH_2-COOH$ (IVb) oder einem funktionellen Derivat, worin Hal für Halogen, wie Chlor, steht, davon in Gegenwart einer starken Säure, wie Schwefelsäure, Polyphosphorsäure, zu Verbindungen der Formel

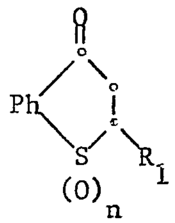

(IVc),

worin n null ist, um. Diese können gewünschtenfalls durch Behandeln mit einem geeigneten Oxidationsmittel, wie m-Chlorperbenzoesäure, S-oxidiert werden, wobei bei Verwendung eines Aequivalents Oxidationsmittel eine SO-Verbindung und bei Umsetzung mit mindestens zwei Mol Oxidationsmittel eine entsprechende $SO_2$-Verbindung erhalten wird.

Die Umsetzung von Verbindungen der Formel (IVc), worin n 0, 1 oder 2 ist, mit Hydroxylamin führt zu entsprechenden Oximen, die anschliessend durch Behandeln mit p-Toluolsulfonylchlorid zu entsprechenden Sulfonylestern der Formel

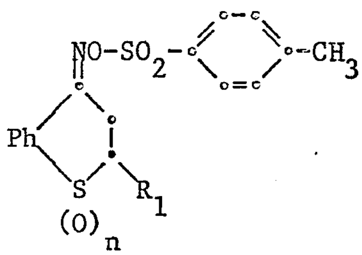

(IVd)

umgesetzt werden können. Die Behandlung dieser Verbindungen der Formel (IVd) mit Natriumethylat und die anschliessende saure Hydrolyse und Neutralisation führt zu Aminen der Formel

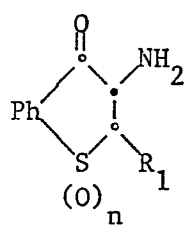

(IVe).

Die Aminogruppe kann im weiteren Verlauf der Reaktionssequenz durch Reaktion mit einer Verbindung der Formel $R-X_1$ (IIb), worin $X_1$ die eingangs angegebene Bedeutung hat, insbesondere einem Säurehalogenid, in die Gruppierung A überführt werden. Aus dieser Umsetzung resultieren die entsprechenden Ausgangsverbindungen der Formel (IV).

Ein weiterer Verfahrensweg zur Herstellung von Verbindungen der Formel (I), worin $R_1$ Wasserstoff darstellt, oder deren Salzen besteht darin, dass in einer Verbindung der Formel

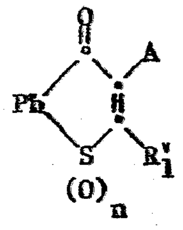

(V),

worin n, Ph und A die angegebenen Bedeutungen haben, und $R_1'$ einen in $R_1$ überführbaren Rest bedeutet, den Rest $R_1'$ in $R_1$ übergeführt wird und man, wenn erwünscht, eine verfahrensgemäss erhältliche freie Verbindung in eine andere freie Verbindung überführt, ein verfahrensgemäss erhältliches Salz in die freie Verbindung oder in ein anderes Salz überführt, eine verfahrensgemäss erhältliche freie Verbindung in ein Salz überführt und/oder gewünschtenfalls ein verfahrensgemäss erhältliches Isomerengemisch in seine Komponenten auftrennt.

In $R_1$ überführbare Reste $R_1'$ sind beispielsweise Carboxy oder Halogen, insbesondere Chlor oder Brom, ferner Iod.

Die Decarboxylierung von Verbindungen der Formel (V), worin $R_1'$ Carboxy darstellt, oder deren Salzen wird üblicherweise unter Erhitzen, beispielsweise in einem Temperaturbereich von etwa 100° bis etwa 300°C, gegebenenfalls in Gegenwart eines Uebergangsmetalles oder einer Legierung davon, z.B. Kupfer oder Kupferbronze, oder einer Base, wie eines basischen Stickstoffheterocyclus, z.B. Pyridin oder Chinolin, oder eines Alkylamins, wie Triniederalkylamins, durchgeführt.

Die reduktive Ueberführung von Verbindungen der Formel (V), worin $R_1'$ Halogen bedeutet, oder deren Salzen erfolgt beispielsweise durch Hydrierung in Gegenwart eines Hydrierungskatalysators, wie eines Elementes der VIII. Nebengruppe des Periodensystems oder eines Derivats davon, z.B. Oxid, wobei der Katalysator gegebenenfalls auf einem geeigneten Trägermaterial, wie Aktivkohle oder Bariumcarbonat, aufgezogen sein kann.

Als Beispiele für solche Katalysatoren sind die üblichen Hydrierungskatalysatoren, insbesondere Raney-Nickel oder Palladium/Kohle, zu nennen. Erforderlichenfalls wird die Hydrierung in Gegenwart einer Säure oder insbesondere einer Base durchgeführt. Entsprechende Säuren sind Protonsäuren, wie Mineralsäuren, z.B. Halogenwasserstoffsäuren, ferner Carbonsäuren, wie Niederalkancarbonsäuren. Als Basen kommen beispielsweise Alkalimetallhydroxide, -carbonate oder -acetate, Amine, wie Niederalkylamine, oder basische Heterocyclen, wie Pyridin oder Chinolin, in Frage.

Ferner kann Halogen $R'_1$ durch Behandeln mit rotem Phosphor und/oder Iodwasserstoffsäure in Wasserstoff $R_1$ überführt werden, z.B. indem auf etwa 100° bis etwa 250°C erwärmt wird.

Die Ausgangsstoffe der Formel (V) werden nach in der Literatur für analoge Reaktionen beschriebenen Verfahren hergestellt.

Zur Herstellung von Verbindungen der Formel (V), worin $R'_1$ Carboxy bedeutet, geht man beispielsweise von Verbindungen der Formel

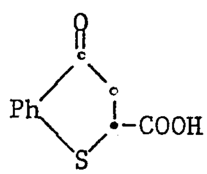

(Va)

aus, führt sie, sofern erwünscht, mit einem geeigneten Oxidationsmittel, wie m-Chlorperbenzoesäure, in die S-Oxide über und setzt diese oder die Verbindungen der Formel (Va) mit Hydroxylamin zu den entsprechenden Oximen um, die anschliessend mit p-Toluolsulfonylchlorid verestert werden, woraus Verbindungen der Formel

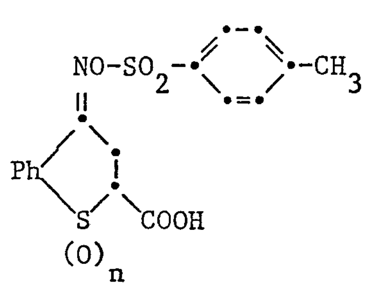

(Vb)

resultieren. Nach Behandlung dieser Verbindungen mit einem der vorstehend aufgeführten Dehydrierungsmittel, wie Trityliumperchlorat, anschliessender Behandlung mit Kaliumethylat, und darauffolgender saurer Hydrolyse und Neutralisation erhält man Amine der Formel

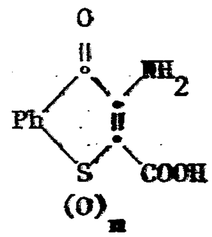

(Vc).

Im folgenden Reaktionsschritt können die Verbindungen der Formel (Vc) durch Umsetzung mit einer Verbindung der Formel $R-X_1$ (IIb), worin $X_1$ die eingangs angegebene Bedeutung hat, insbesondere einem Säurehalogenid, in die entsprechenden Ausgangsstoffe der Formel (V) überführt werden, worin $R_1'$ für Carboxy steht.

Ausgangsverbindungen der Formel (V), worin $R_1'$ Halogen, insbesondere Chlor oder Brom, ferner Iod, bedeutet, können folgendermassen hergestellt werden. Man geht beispielsweise von Verbindungen der Formel

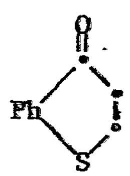

(Ve)

aus und setzt sie mit Isopentylnitrit in Kaliumhydroxidlösung um, wobei entsprechende Verbindungen der Formel

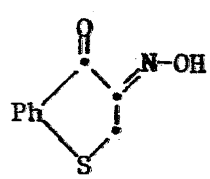

(Vf)

anfallen. Diese werden wiederum mit einer Halogenwasserstoffsäure, insbesondere Chlor- oder Bromwasserstoffsäure,behandelt. Daraus resultieren entsprechende Verbindungen der Formel

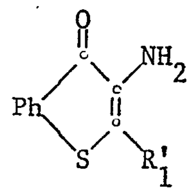

(Vg),

worin $R_1'$ Halogen bedeutet.

Diese können, sofern entsprechende Verbindungen der Formel (V) gewünscht werden, worin n 1 oder 2 bedeutet, nach Schutz der Aminogruppe, mit einem geeigneten Oxidationsmittel, wie m-Chlorperbenzoesäure, entsprechend S-oxidiert werden. Nach Abspaltung der Aminoschutzgruppe kann im folgenden Reaktionsschritt die Aminogruppe in Verbindungen der Formel (Vg) oder deren S-Oxiden durch Umsetzung mit einer Säure der Formel R-COOH (Vf) oder einem reaktionsfähigen Derivat, z.B. Säurehalogenid, davon in die gewünschte Gruppe A überführt werden.

Die erfindungsgemässen Verbindungen der Formel I können ferner hergestellt werden, indem man eine Verbindung der Formel

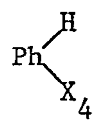

(VI),

worin Ph die angegebene Bedeutung hat und $X_4$ eine Gruppe der Formel $-S(O)_n-C(R_1)=C(A)-X_5$, bedeutet, in der $X_5$ gegebenenfalls funktionell abgewandeltes Carboxy bedeutet, oder ein Salz davon cyclisiert und, wenn erwünscht, eine verfahrensgemäss erhältliche freie Verbindung in eine andere freie Verbindung überführt, ein verfahrensgemäss erhältliches Salz in die freie Verbindung oder in ein anderes Salz überführt, eine verfahrensgemäss erhältliche freie Verbindung in ein Salz überführt und/oder gewünschtenfalls ein verfahrensgemäss erhältliches Isomerengemisch in seine Komponenten auftrennt.

Funktionell abgewandeltes Carboxy bedeutet in erster Linie verestertes oder anhydridisiertes Carboxy. Verestertes Carboxy ist vorzugsweise mit einem Niederalkanol verestertes Carboxy, wie Niederalkoxycarbonyl, während anhydridisiertes Carboxy beispielsweise mit einer

Niederalkancarbonsäure oder einer gegebenenfalls substituierten Benzoesäure oder in erster Linie mit einer Halogenwasserstoffsäure oder einem Kohlensäure-halogenid-niederalkylhalbester anhydridisiertes Carboxy, wie Niederalkanoyloxy- bzw. Benzoyloxycarbonyl, Halogencarbonyl oder Niederalkoxycarbonyloxycarbonyl, bedeutet.

Die Cyclisierung erfolgt intramolekular und kann insbesondere in der aus der Literatur für analoge Reaktionen bekannten Weise, erforderlichenfalls in Gegenwart eines Kondensationsmittels und/oder bei erhöhter Temperatur, z.B. der Siedetemperatur des Lösungsmittels, durchgeführt werden.

Als Kondensationsmittel eignen sich beispielsweise starke anorganische oder organische Protonsäuren, wie Mineralsäuren, z.B. Halogenwasserstoffsäuren, Schwefelsäure oder Polyphosphorsäure, wie Sulfonsäuren, z.B. Alkan- oder gegebenenfalls substituierte Benzolsulfonsäuren, beispielsweise p-Toluolsulfonsäure, oder wie organische Carbonsäuren, z.B. Niederalkancarbonsäuren, beispielsweise Essigsäure. Weiterhin können für die Cyclisierung Lewis-Säuren verwendet werden, z.B. Verbindungen von Elementen der dritten und fünften Hauptgruppe sowie der zweiten und achten Nebengruppe des Periodensystems. In Betracht kommen vor allem Halogenide des Bors, Aluminiums, Zinns, Antimons und Eisens, wie Bortrifluorid, Aluminiumchlorid, Zinn-(IV)-chlorid, Zinkchlorid und Eisen-(III)-chlorid.

Die Ausgangsstoffe sind bekannt oder können, sofern sie neu sind, nach an sich bekannten Methoden hergestellt werden.

So kann man beispielsweise Ausgangsstoffe der Formel VI herstellen, worin $X_4$ eine Gruppe der Formel $-S(O)_n-C(R_1)=C(A)-X_5$ und n null bedeuten, indem man Verbindungen der Formel

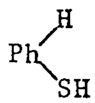

(VIa)

mit Verbindungen der Formel

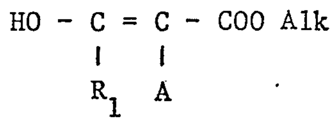

(VIb),

worin Alk Niederalkyl bedeutet, mit Hilfe eines Kondensationsmittels, wie Polyphosphorsäure, zu den entsprechenden Verbindungen der Formel (VI) kondensiert.

Die Verbindungen der Formel (VIb) können durch Umsetzung von Verbindungen der Formel $HO-C(R_1)=C(NH_2)-COO$ Alk (VIc) oder deren Tautomeren mit Verbindungen der Formel R-COOH (VId) oder deren funktionellen Derivaten, insbesondere mit entsprechenden Säurehalogeniden, enthalten werden.

Die Verbindungen der Formel (VIc) ihrerseits sind bekannt oder können nach an sich bekannten Verfahren hergestellt werden. Beispielsweise sind sie zugänglich, indem man Verbindungen der Formel $R_1-CO-CH_2-CO-Alk$ (VId) in Essigsäure mit Natriumnitrit behandelt und in resultierenden Verbindungen der Formel $R_1-CO-C(=NOH)-CO-Alk$ (VIe) die Hydroxyiminogruppe mit Wasserstoff in Gegenwart von Salzsäure reduziert.

Ein weiterer Weg zur Herstellung der erfindungsgemässen Verbindungen der Formel (I) besteht darin, dass man in einer Verbindung der Formel

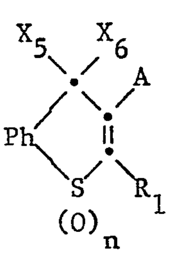

(VII),

worin A, $R_1$, n und Ph die angegebenen Bedeutungen haben und $X_5$ und $X_6$ gemeinsam in die Oxogruppe überführbare Reste darstellen, oder einem Salz davon $X_5$ und $X_6$ in die Oxogruppe überführt und, wenn erwünscht, eine verfahrensgemäss erhältliche freie Verbindung in eine andere freie Verbindung überführt, ein verfahrensgemäss erhältliches Salz in die freie Verbindung oder in ein anderes Salz überführt, eine verfahrensgemäss erhältliche freie Verbindung in ein Salz überführt und gewünschtenfalls ein verfahrensgemäss erhältliches Isomerengemisch in seine Komponenten auftrennt.

In die Oxogruppe überführbare Reste $X_5$ und $X_6$ können gemeinsam funktionell abgewandeltes Oxo bilden, wie Thioxo, gegebenenfalls substituiertes Imino, Niederalkylendioxy, Niederalkylendithioxy oder jeweils unabhängig voneinander Niederalkoxy oder Niederalkylthio. $X_5$ und $X_6$ können gemeinsam hydrolytisch in die Oxogruppe überführt werden. Dabei wird erforderlichenfalls in Gegenwart eines katalytischen Mittels, wie einer Protonsäure, in einem inerten Lösungsmittel und unter Erwärmen, z.B. auf etwa 50° bis etwa 150°C gearbeitet. Als geeignete Protonsäuren kommen beispielsweise Mineralsäuren, wie Halogenwasserstoffsäuren oder Schwefelsäure, aliphatische Carbonsäuren, wie Niederalkancarbonsäuren, oder Sulfonsäuren, wie gegebenenfalls substituierte Benzolsulfonsäuren, in Frage.

Eine substituierte Iminogruppe kann beispielsweise durch einen aliphatischen oder aromatischen Rest, wie Niederalkyl oder Phenyl, oder durch einen Acylrest, der sich von einer Carbonsäure oder einem Halbester der Kohlensäure ableitet, wie Niederalkanoyl, Benzoyl oder Niederalkoxycarbonyl, substituiert sein.

Das Ausgangsmaterial der Formel (VII), worin $X_5$ und $X_6$ gemeinsam Thioxo bilden, erhält man beispielsweise durch Umsetzung von Verbindungen der Formel

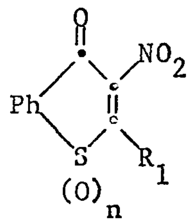

(VIIa)

mit Phosphorpentasulfid in Gegenwart von Natriumhydrogencarbonat oder mit Siliziumdisulfid unter Erwärmen. Entsprechende Thioketale können beispielsweise durch Reaktion von Verbindungen der Formel (VIIa) mit Mercaptoverbindungen, wobei in Gegenwart von Salzsäure gearbeitet wird, O-Acetale analog durch Umsetzung mit Alkoholen und p-Toluolsulfonsäure, erhalten werden. Zu entsprechenden Iminen gelangt man durch Reaktion von Verbindungen der Formel (VIIa) mit Ammoniak bzw. primären Aminen in Gegenwart von Salzsäure, Natriumhydroxid oder Zinkchlorid. Anschliessend werden die so erhältlichen Verbindungen zu Verbindungen der Formel

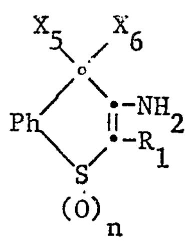

(VIIb)

oder deren Salze reduziert und durch Umsetzung mit Verbindungen der Formel $R-X_1$ (IIb), worin $X_1$ die eingangs angegebenen Bedeutungen hat, z.B. Carbonsäurehalogeniden, in die entsprechenden Verbindungen der Formel (VII) übergeführt.

Die vor und nachstehend beschriebenen Umsetzungen werden nach an sich bekannten Verfahren durchgeführt, beispielsweise in Ab- oder üblicherweise Anwesenheit eines geeigneten Lösungs- bzw. Verdünnungsmittels oder eines Gemisches derselben, wobei man je nach Verfahrensweise unter Kühlen, bei Raumtemperatur oder unter Erwärmen, z.B. in einem Temperaturbereich von etwa -20° bis zur Siedetemperatur des Reaktionsmediums, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial, das für die Herstellung der Verbindung der Formel I und ihrer Salze entwickelt wurde, ist zum Teil bekannt bzw. kann nach an sich bekannten Methoden, z.B. analog der vor- und nachstehend beschriebenen Verfahrensvarianten, hergestellt werden.

Eine erfindungsgemäss oder anderweitig erhältliche Verbindung der Formel (I) kann in an sich bekannter Weise in eine andere Verbindung der Formel (I) umgewandelt werden.

So kann man beispielsweise Verbindungen der Formel (I), worin 1,2-Phenylen Ph Wasserstoff aufweist, halogenieren, wie chlorieren oder bromieren, indem entsprechende Verbindungen in einem Temperaturbereich von etwa 0°C bis etwa 150°C, mit einem geeigneten Halogenierungsmittel, wie Chlor oder Brom, in einem unpolaren Lösungsmittel, wie Methylenchlorid, gegebenenfalls in Gegenwart einer Lewis-Säure, umgesetzt werden.

Ferner kann der Benzoteil des Ringsystems beispielsweise mit einem Niederalkanol bzw. einem Niederalkylhalogenid oder einem Phosphorsäureniederalkylester in Gegenwart von Lewis-Säuren alkyliert werden. In einer Verbindung der Formel (I), worin 1,2-Phenylen Ph, z.B. Brom enthält, kann beispielsweise das Brom durch Umsetzung mit einem Niederalkylbromid in Gegenwart eines Alkalimetalls, insbesondere Lithium, durch Niederalkyl ersetzt werden.

Enthält 1,2-Phenylen Ph als Substituenten Hydroxy, so lässt sich dieses nach an sich bekannter Weise verethern. Die Umsetzung mit einer Alkoholkomponente, z.B. mit einem Niederalkanol, wie Ethanol, in Gegenwart von Säuren, z.B. Mineralsäure, wie Schwefelsäure, oder von Dehydratisierungsmitteln, wie Dicyclohexylcarbodiimid, führt zu Niederalkoxy. Umgekehrt kann man Ether in Phenole spalten, indem man die Etherspaltung mittels Säuren, wie Mineralsäuren, z.B. Halogenwasserstoffsäuren, wie Bromwasserstoffsäure, oder wie Lewissäuren, z.B. Halogeniden von Elementen der 3. Hauptgruppe, wie Bortribromid, oder mittels eines Pyridinium-hydrohalogenids, z.B. Pyridin-hydrochlorid, oder Thiophenol durchführt.

Weiter lässt sich Hydroxy in Niederalkanoyloxy umwandeln, beispielsweise durch Umsetzung mit einer erwünschten Niederalkancarbonsäure, wie Essigsäure, oder einem reaktionsfähigen Derivat davon, beispielsweise in Gegenwart einer Säure, wie eine Protonsäure, z.B. Chlor-, Bromwasserstoff-, Schwefel-, Phosphor- oder einer Benzolsulfonsäure, in Gegenwart einer Lewissäure, z.B. von Bortrifluorid-Etherat, oder in Gegenwart eines wasserbindenden Mittels, wie Dicyclohexylcarbodiimid. Umgekehrt kann verestertes Hydroxy, z.B. durch Basen-Katalyse, zu Hydroxy solvolysiert werden.

Freie und veresterte Carboxygruppe R kann man ineinander, beispielsweise eine freie Carboxygruppe in üblicher Weise in eine veresterte Carboxylgruppe R überführen, vorzugsweise durch Umsetzung mit einem entsprechenden Alkohol oder einem reaktionsfähigen Derivat, wie einem Carbon-, Phosphorig-, Schweflig- oder Kohlensäureester, z.B. einem Niederalkancarbonsäureester, Triniederalkylphosphit, Diniederalkylsulfit oder dem Pyrocarbonat, oder einem Mineralsäure- oder Sulfonsäureester, z.B. dem Chlor- oder Bromwasserstoffsäure- oder Schwefelsäure-, Benzolsulfonsäure-, Toluolsulfonsäure- oder Methansulfonsäureester, des entsprechenden Alkohols oder einem davon abgeleiteten Olefin.

Die Umsetzung mit dem entsprechenden Alkohol erfolgt vorteilhaft in Gegenwart eines sauren Katalysators, wie einer Protonensäure, z.B. von Chlor- oder Bromwasserstoff-, Schwefel-, Phosphor-, Bor-, Benzolsulfon- und/oder Toluolsulfonsäure, oder einer Lewissäure, z.B. von

Bortrifluorid-Etherat, in einem inerten Lösungsmittel, insbesondere einem Ueberschuss des eingesetzten Alkohols und erforderlichenfalls in Gegenwart eines wasserbindenden Mittels und/oder unter destillativer, z.B. azeotroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester, beispielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Ueberschuss des eingesetzten Alkoholderivates oder des entsprechenden Alkohols, erforderlichenfalls unter, z.B. azeotroper, Abdestillation des Reaktionswassers. Ausgehend von einem Mineralsäure- oder Sulfonsäureester setzt man die zu veresternde Säure vorteilhaft in Form eines Salzes, z.B. des Natrium-, Kalium- oder Calciumsalzes, z.B. in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triethylamin oder Pyridin, erforderlichenfalls in einem inerten Lösungsmittel, wie einer der vorstehenden tertiären Stickstoffbasen oder eines polaren Lösungsmittels, z.B. in Dimethylformamid und/oder bei erhöhter Temperatur um.

Die Umsetzung mit einem Olefin kann beispielsweise in Gegenwart eines sauren Katalysators, z.B. einer Lewissäure, z.B. von Bortrifluorid, einer Sulfonsäure, z.B. von Toluolsulfonsäure, oder vor allem eines basischen Katalysators, z.B. von Natrium- oder Kaliumhydroxid, vorteilhaft in einem inerten Lösungsmittel, wie einem Ether, z.B. in Diethylether oder Tetrahydrofuran, erfolgen.

Die vorstehend beschriebenen Umwandlungen freier in veresterte Carboxylgruppen R können aber auch so durchgeführt werden, dass man eine Verbindung der Formel I, worin R Carboxyl ist, zunächst in üblicher Weise in ein reaktionsfähiges Derivat, beispielsweise mittels eines Halogenids des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol oder Amin in einen reaktionsfähigen Ester, d.h. Ester mit elektronenanziehenden Strukturen, wie den Ester mit Phenol, Thiophenol, p-Nitrophenol oder Cyanmethylalkohol, oder ein reaktives Amid, z.B. das von Imidazol oder 3,5-Dimethylpyrazol abgeleitete Amid, überführt und das erhaltene reaktionsfähige Derivat dann in üblicher Weise, z.B. wie nachstehend für die Umesterung bzw. gegenseitige Umwandlung veresterter Carboxylgruppen R beschrieben, mit einem entsprechenden Alkohol zu der gewünschten Gruppe R umsetzt.

Eine veresterte Carboxylgruppe R kann ferner in üblicher Weise, z.B. durch Umsetzung mit einem entsprechenden Metallalkoholat, z.B. dem Natrium- oder Kaliumalkoholat des entsprechenden Alkohols, oder mit diesem selbst in Gegenwart eines Katalysators, beispielsweise einer starken Base, z.B. von Natrium- oder Kaliumhydroxid, oder einer starken Säure, wie einer Mineralsäure, z.B. von Salzsäure, Schwefelsäure oder Phosphorsäure, oder einer organischen Sulfonsäure, z.B. von p-Toluolsulfonsäure, oder einer Lewissäure, z.B. von Bortrifluorid-Aetherat, zu einer anderen veresterten Carboxylgruppe R umgesetzt werden.

Eine amidierte Carboxylgruppe R kann in üblicher Weise, z.B. durch Hydrolyse in Gegenwart eines Katalysators, beispielsweise einer starken Base, wie eines Alkalimetall- oder Erdalkalimetallhydroxides oder -carbonates, z.B. von Natrium- oder Kaliumhydroxid oder -carbonat, oder einer starken Säure, wie einer Mineralsäure, z.B. von Salzsäure, Schwefelsäure oder Phosphorsäure, in die freie Carboxylgruppe R umgewandelt werden.

Bedeutet n der Formel (I) null, so kann das Schwefelatom auf übliche Weise zu entsprechendem Sulfinyl bzw. Sulfonyl oxidiert werden. Als geeignetes Oxidationsmittel für die Oxidation zur Sulfoxidstufe kommen beispielsweise anorganische Persäuren, wie Persäuren von Mineralsäuren, z.B. Perjodsäure oder Perschwefelsäure, organische Persäuren, wie entsprechende Percarbon- oder Persulfonsäuren, z.B. Perameisen-, Peressig-, Trifluorperessig- bzw. m-Chlor-perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Uebergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.

Die Oxidation zur Sulfonstufe kann man mit Distickstofftetroxid als Katalysator in Gegenwart von Sauerstoff bei tiefen Temperaturen entsprechend durchführen, ebenso wie die direkte Oxidation des Thio zum Sulfonyl. Jedoch setzt man hierbei üblicherweise das Oxidationsmittel im Ueberschuss ein. Entsprechend kann die Oxidation von durch einen aliphatischen Rest substituiertes Mercapto bzw. Sulfeno als Substituenten von Ph durchgeführt werden.

In Verbindungen der Formel I, worin n 1 oder 2 bedeutet, kann die Sulfinyl- bzw. Sulfonylgruppe zu Thio reduziert werden. Entsprechend kann die Reduktion von durch einen aliphatischen Rest substituiertes Sulfonyl bzw. Sulfinyl als Substituenten von Ph zu entsprechendem Sulfenyl bzw. Mercapto durchgeführt werden. Als Reduktionsmittel eignen sich beispielsweise katalytisch aktivierter Wasserstoff,

wobei Edelmetalle bzw. Oxide, wie Palladium, Platin oder Rhodium bzw. deren Oxide, verwendet werden, gegebenenfalls auf geeignetem Trägermaterial, wie Aktivkohle oder Bariumsulfat, aufgezogen. Weiterhin kommen reduzierende Metallkationen, wie Zinn II-, Blei II-, Kupfer I-, Mangan II-, Titan II-, Vanadium II-, Molybdän III- oder Wolfram III-Verbindungen, Halogenwasserstoff, wie Chlor-, Brom- oder Iodwasserstoff, Hydride, wie komplexe Metallhydride, z.B. Lithiumaluminium-, Natriumbor-, Tributylzinnhydrid, Phosphorverbindungen, wie Phosphorhalogenide, z.B. Phosphortrichlorid, -bromid, Phosphorpentachlorid oder Phosphoroxichlorid, Phosphine, wie Triphenylphosphin, oder Phosphorpentasulfid-Pyridin, oder Schwefelverbindungen, wie Mercaptane, Thiosäuren, wie Thiophosphorsäuren oder Dithiocarbonsäuren, Dithionit oder Schwefel-Komplexe, wie Iod-Pyridin-Schwefeldioxidkomplex, in Frage.

Enthalten die Verbindungen der Formel (I) ungesättigte Reste, wie Niederalkenylgruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at. und bei Temperaturen zwischen etwa -80 bis etwa 200°C, vor allem zwischen Raumtemperatur und etwa 100°C durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Salze von Verbindungen der Formel (I) mit salzbildenden Gruppen können in an sich bekannter Weise hergestellt werden. So kann man Salze von Verbindungen der Formel (I) mit sauren Gruppen z.B. durch Behandeln mit Metallverbindungen, wie Alkalimetallalkoholaten oder Alkalimetallsalzen von geeigneten organischen Carbonsäuren, z.B. dem Natriumsalz der α-Ethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet.

Salze können in üblicher Weise in die freien Verbindungen übergeführt werden, Metall- und Ammoniumsalze z.B. durch Behandeln mit geeigneten Säuren.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt und/oder einen Ausgangsstoff in Form eines Salzes, Isomeren und/oder Racemates bzw. Antipoden verwendet oder insbesondere unter den Reaktionsbedingungen bildet.

Infolge der engen Beziehungen zwischen der neuen Verbindungen in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemisch, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Ueberführung in diastereomere Salze oder Ester, z.B. durch Umsetzung eines sauren Endstoffes mit einer mit der racemischen Säure Salze bildenden optisch aktiven Base oder einer optisch aktiven Carbonsäure oder einem reaktiven Derivat davon, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann, zerlegt. Vorteilhaft isoliert man das wirksamere Enantiomere.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, ihre Verwendung, z.B. als Arzneimittelwirkstoffe, Formulierungsverfahren und Verfahren zu ihrer Herstellung bilden ebenfalls einen Gegenstand der Erfindung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen

oder rektalen, und parenteralen Verabreichung sowie zur Inhalation durch/an Warmblüter(n), wobei der pharmakologische Wirkstoff allein oder zusammen mit einem pharmazeutisch anwendbaren Trägermaterial enthalten ist. Die tägliche Dosis des Wirkstoffes hängt von dem Alter und dem individuellen Zustand, sowie von der Applikationsweise ab.

Die neuen pharmazeutischen Präparate enthalten z.B. von etwa 10% bis etwa 90 %, vorzugsweise von etwa 20% bis etwa 60% des Wirkstoffs. Erfindungsgemässe pharmazeutische Präparate sind z.B. solche in Aerosol-, oder Sprayform oder in Dosiseinheitsformen wie Dragées, Tabletten, Kapseln oder Suppositorien, ferner Augen- oder Nasentropfen sowie Ampullen. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister und Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Inhalationspräparate für die Behandlung der Atemwege durch nasale oder buccale Verabreichung sind z.B. Aerosole oder Sprays, welche den pharmazeutischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe, wie flüssige oder feste, nichtionische oder anionische oberflächenaktive Mittel und/oder feste Verdünnungsmittel. Präparate, in welchen der pharmazeutischen Wirkstoff in Lösung vorliegt, enthalten ausser diesen ein geeignetes Treibmittel, ferner, falls notwendig, ein zusätzliches Lösungsmittel und/oder einen Stabilisator. Anstelle des Treibgases kann auch Druckluft verwendet werden.

Die erfindungsgemässen pharmazeutischen Inhalationspräparate sind beispielsweise Aerosol-, vorzugsweise Feststoffaerosolbildende Gemische, d.h. Gemische, die mittels ihres eigenen Dampfdruckes oder des Druckes eines komprimierten Gases, wie Druckluft, Lachgas oder Kohlendioxid, inhalierbare Aerosole, vorzugsweise inhalierbare Feststoffaerosole bilden können.

Die Erfindung betrifft ebenfalls die Verwendung der neuen Verbindungen sowie ihrer Salze als pharmakologisch aktive Verbindungen, insbesondere als Antiallergika, vorzugsweise in Form von pharmazeutischen Präparaten und zur Anwendung in einem Verfahren zur Behandlung allergischer Erkrankungen. Die Dosis, die z.B. einem Warmblüter von etwa 70 kg verabreicht wird, beträgt, bei oraler Applikation, von etwa 100 mg bis etwa 1000 mg, vorzugsweise von etwa 250 bis etwa 750 mg.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

Beispiel 1: Zu einer Lösung von 5,05 g (0,0216 Mol) 3-Amino-6-(n-butyl)-4H-1-benzothiopyran-4-on und 2,4 g (0,024 Mol) Triethylamin in 325 ml Chloroform werden 2,94 g (0,024 Mol) Oxalsäuremonomethylesterchlorid zugegeben, wobei das Reaktionsgemisch sich auf 30° erwärmt. Anschliessend wird noch eine Stunde bei Raumtemperatur gerührt. Dann dampft man zur Trockne ein, verrührt den kristallinen Rückstand mit Ethanol, nutscht ab, wäscht zunächst mit Ethanol und dann mit Ether. Nach dem Trocknen erhält man das N-[6-(n-Butyl)-4H-1-benzothiopyran-4-on-3-yl]-oxalsäureamid-methylester vom Smp. 192-193,5°.

Das Ausgangsmaterial kann wie folgt hergestellt werden: Zu einer Lösung von 34,4 g (0,86 Mol) Natriumhydroxid in 565 ml Wasser werden bei etwa 20° 143 g (0,86 Mol) p-(n-Butyl)-thiophenol zugegeben. Anschliessend werden noch 131 ml 2N Natronlauge zugegeben. Dann wird innerhalb von 30 Minuten bei maximal 30° eine Lösung von 131,5 g (0,86 Mol) 3-Brompropionsäure in 334 ml Wasser und 52,4 g (0,49 Mol) wasserfreiem Natriumcarbonat zugetropft und das Reaktionsgemisch 3 Stunden bei 40-45° gerührt. Nach dem Abkühlen wird zunächst mit Ether extrahiert. Dann giesst man die wässrige Phase in 300 ml konz. Salzsäure. Das ausgeschiedene Oel wird in Methylenchlorid aufgenommen, der Methylenchloridauszug mit wasserfreiem Magnesiumsulfat getrocknet, eingedampft und der verbleibende Rückstand mit wenig Petrolether verrührt. Man lässt noch 3 Stunden im Eisbad stehen und nutscht dann das kristalline Produkt ab. Man erhält so die 3-[4-(n-Butyl)-phenylthio]-propionsäure vom Smp. 58-59°.

129,2 g (0,542 Mol) 3-[4-(n-Butyl)-phenylthio]-propionsäure werden portionsweise zu 685 ml konz. Schwefelsäure gegeben. Die entstandene Lösung wird 1 Stunde bei Raumtemperatur gerührt und dann auf Eis gegossen. Die organische Phase wird anschliessend in Methylenchlorid aufgenommen. Der Methylenchloridauszug wird einmal mit Wasser und einmal mit 2N Natronlauge gewaschen, mit wasserfreiem Magnesiumsulfat getrocknet und eingedampft. Man erhält so das 6-(n-Butyl)-2,3-dihydro-4H-1-benzothiopyran-4-on in Form eines gelben Oels.

Zu einer Lösung von 30,6 g (0,139 Mol) 6-(n-Butyl)-2,3-dihydro-4H-1-benzothiopyran-4-on und 18 g (0,154 Mol) Isopentylnitrit in 85 ml Ethanol wird innerhalb von 45 Minuten bei 0-5° eine Lösung von 8,2 g (0,12 Mol) Kaliumhydroxid in 135 ml Ethanol zugetropft. Anschliessend wird noch 2 Stunden bei 0-5° gerührt und das Reaktionsgemisch zur Trockne eingedampft. Das zurückbleibende Kaliumsalz des 6-(n-Butyl)-3-hydroxyimino-4H-1-benzothiopyran-4-ons (Oel) wird ohne Reinigung weiter umgesetzt.

Zur einer Lösung von 56 g von rohem 6-(n-Butyl)-3-hydroxyimino-4H-1-benzothiopyran-4-on-Kaliumsalz in 250 ml Eisessig werden innerhalb von 20 Minuten bei etwa 20° 60 ml konz. Salzsäure zugetropft. Anschliessend rührt man noch 1 Stunde bei 0-5° und dampft dann das Reaktionsgemisch zur Trockne ein. Der Rückstand wird mit Methylenchlorid und Natronlauge verrührt, die organische Phase abgetrennt, zweimal mit Wasser gewaschen und mit wasserfreiem Magnesiumsulfat getrocknet. Anschliessend wird filtriert, die Lösung auf etwa 100 ml eingeengt und über eine mit Kieselgel gefüllte Säule filtriert. Man eluiert mit Methylenchlorid. Die einzelnen Fraktionen, je etwa 250 ml, werden mit Dünnschichtchromatographie überprüft. Die Fraktionen 5-11 werden vereinigt, das Lösungsmittel abdestilliert, das verbleibende Oel mit Petrolether verrieben und das auskristallisierte Produkt abgenutscht. Man erhält so das 3-Amino-2-chlor-6-(n-butyl)-4H-1-benzothiopyran-4-on vom Smp. 84,5-86°.

Eine Lösung von 7,7 g (0,029 Mol) 3-Amino-6-(n-butyl)-2-chlor-4H-1-benzothiopyran-4-on und 3,2 g Triethylamin in 160 ml Ethanol wird bei 20-25° in Gegenwart von 0,8 g 10%iger Palladiumkohle bei Normaldruck hydriert. Der Katalysator wird abfiltriert, das Filtrat zur Trockne eingedampft, der Rückstand in Methylenchlorid gelöst und über eine mit Kieselgel gefüllte Säule filtriert, wobei mit Methylenchlorid eluiert wird. Aus den Hauptfraktionen erhält man, nach Verrühren mit Petrolether, reines 3-Amino-6-(n-butyl)-4H-1-benzothiopyran-4-on vom Smp. 75-76°.

Beispiel 2: 6,15 g (0,0193 Mol) N-[6-(n-Butyl)-4H-1-benzothiopyran-4-on-3-yl]-oxamid-methylester werden mit einer Lösung von 22,6 ml 1N-Natronlauge in 147 ml Ethanol und 970 ml Wasser 2 Stunden bei 70° gerührt. Dann wird von einer geringen Trübung abfiltriert und das Filtrat mit Salzsäure angesäuert, wobei ein kristallines Produkt ausfällt. Nach dem Erkalten werden die Kristalle abgenutscht und zuerst mit Wasser, dann mit Ethanol und anschliessend mit Ether gewaschen. Nach dem Trocknen erhält man das N-[6-(n-Butyl)-4H-1-benzothiopyran-4-on-3-yl]-oxalsäuremonoamid vom Smp. 208° (Zers.).

Beispiel 3: In analoger Weise wie in Beispiel 1 können hergestellt werden:

N-(4H-1-benzothiopyran-4-on-3-yl)-oxamid-methylester vom Smp. 260°,

N-(6-Chlor-4H-1-benzothiopyran-4-on-3-yl)-oxamid-methylester vom Smp. 305°,

N-(6-Methyl-4H-1-benzothiopyran-4-on-3-yl)-oxamid-methylester vom Smp. 238-240°.

Beispiel 4: In analoger Weise wie in Beispiel 1 beschrieben können hergestellt werden:
N-(6,7-Trimethylen-4H-1-benzothiopyran-4-on-3-yl)-oxamid-methylester vom Smp. 255-256° und
N-(5,6-Trimethylen-4H-1-benzothiopyran-4-on-3-yl)-oxamid-methylester vom Smp. 265,5-266,5°.

Das Ausgangsmaterial kann wie folgt hergestellt werden:
In analoger Weise wie in Beispiel 1 beschrieben erhält man ausgehend von Indan-5-thiol die 3-(3,4-Trimethylen-phenylthio)-propionsäure (braunes Oel), welche durch Behandeln mit konz. Schwefelsäure ein Isomerengemisch ergibt. Dieses besteht überwiegend aus 6,7-Trimethylen-2,3-dihydro-4H-1-benzothiopyran-4-on und ferner aus 5,6-Trimethylen-2,3-dihydro-4H-1-benzothiopyran-4-on, Smp. 61,5-63,5°. Dieses Isomerengemisch wird mit Isopentylnitrit oximiert und anschliessend mit konz. Salzsäure behandelt. Nach Chromatographie der Reaktionsprodukte erhält man 3-Amino-2-chlor-6,7-trimethylen-4H-1-benzothiopyran-4-on vom Smp. 173-174° und 3-Amino-2-chlor-5,6-trimethylen-4H-1-benzothiopyran-4-on vom Smp. 133-134°. Beide Isomeren werden in analoger Weise wie in Beispiel 1 beschrieben, hydriert. Man erhält so das 3-Amino-6,7-trimethylen-4H-1-benzothiopyran-4-on, Smp. 145-146°, und 3-Amino-5,6-trimethylen-4H-1-benzothiopyran-4-on, Smp. 172-174°.

Beispiel 5: In analoger Weise wie in Beispiel 1 beschrieben erhält man:
N-(5,7-Dimethyl-4H-1-benzothiopyran-4-on-3-yl)-oxamid-methylester vom Smp. 208-209°.

Das Ausgangsmaterial kann wie folgt hergestellt werden:
In analoger Weise wie in Beispiel 1 beschrieben erhält man ausgehend von 3,5-Dimethyl-thiophenol die 3-(3,5-Dimethyl-phenylthio)-propionsäure vom Smp. 69-70°, welche über das 5,7-Dimethyl-2,3-dihydro-

4H-1-benzothiopyran-4-on Smp. 68-69° in das Kaliumsalz des 3-Hydroxyimino-5,7-dimethyl-3,4-dihydro-4H-1-benzothiopyran-4-ons übergeführt wird. Dieses ergibt, nach Behandlung mit konz. Salzsäure, das 3-Amino-2-chlor-5,7-dimethyl-4H-1-benzothiopyran-4-on, Smp. 135,5-136,5°, welches nach Hydrierung das 3-Amino-5,7-dimethyl-4H-1-benzothiopyran-4-on vom Smp. 117,5-118,5° ergibt.

Beispiel 6: In analoger Weise wie in Beispiel 1 beschrieben erhält man:
N-(6-Ethyl-4H-1-benzothiopyran-4-on-3-yl)-oxamid-methylester vom Smp. 203,5-205°.

Das Ausgangsmaterial kann wie folgt hergestellt werden:
In analoger Weise wie in Beispiel 1 beschrieben erhält man ausgehend von 4-Ethyl-thiophenol die 3-(4-Ethyl-phenylthio)-propionsäure vom Smp. 59-60°C, welche in das 6-Ethyl-2,3-dihydro-4H-1-benzothiopyran-4-on (rotes Oel) übergeführt wird. Dieses wird mit Isopentylnitrit oximiert und das erhaltene rohe Kaliumsalz des 6-Ethyl-3-hydroxyimino-2,3-dihydro-4H-1-benzothiopyran-4-ons mit konz. Salzsäure in das 3-Amino-2-chlor-6-ethyl-4H-1-benzothiopyran-4-on, Smp. 79-81°, übergeführt. Nach der Hydrierung erhält man das 3-Amino-6-ethyl-4H-1-benzothiopyran-4-on vom Smp. 106-107°.

Beispiel 7: In analoger Weise wie in Beispiel 2 beschrieben erhält man: N-(4H-1-Benzothiopyran-4-on-3-yl)-oxalsäuremonoamid vom Smp. 220 (Zers.),
N-(6-Chlor-4H-1-benzothiopyran-4-on-3-yl)-oxalsäuremonoamid vom Smp. 245° (Zers.),
N-(6-Methyl-4H-1-benzothiopyran-4-on-3-yl)-oxalsäuremonoamid vom Smp. 222° (Zers.),
N-(6,7-Trimethylen-4H-1-benzothiopyran-4-on-3-yl)-oxalsäuremonoamid-monohydrat vom Smp. 223° (Zers.),

N-(5,6-Trimethylen-4H-1-benzothiopyran-4-on-3-yl)-oxalsäuremonoamid vom Smp. 225° (Zers.),
N-(5,7-Dimethyl-4H-1-benzothiopyran-4-on-3-yl)-oxalsäuremonoamid vom Smp. 217° (Zers.),
N-(6-Ethyl-4H-1-benzothiopyran-4-on-3-yl)-oxalsäuremonoamid vom Smp. 196° (Zers.).

Beispiel 8: 10 g (0,046 Mol) 3-Amino-6,7-trimethylen-4H-1-benzothiopyran-4-on und 10,5 g (0,138 Mol) Glykolsäure werden 1 Stunde bei 120° erhitzt. Nach dem Erkalten wird das Reaktionsgemisch mit Wasser verrührt, das kristalline Produkt abgenutscht, mit Wasser gewaschen und getrocknet. Nach dem Umkristallisieren aus Eisessig erhält man reines N-(6,7-Trimethylen-4H-1-benzothiopyran-4-on-3-yl)-2-hydroxy-essigsäuremonoamid vom Smp. 186,5-188°. In analoger Weise erhält man: N-(4H-1-Benzothiopyran-4-on-3-yl)-2-hydroxy-essigsäuremonoamid vom Smp. 159-161°,
N-(6-Chlor-4H-1-benzothiopyran-4-on-3-yl)-2-hydroxy-essigsäuremonoamid vom Smp. 215-217°,
N-(6-Methyl-4H-1-benzothiopyran-4-on-3-yl)-2-hydroxy-essigsäuremonoamid vom Smp. 222° (Zers.)
N-(5,6-Trimethylen-4H-1-benzothiopyran-4-on-3-yl)-2-hydroxy-essigsäuremonoamid vom Smp. 225° (Zers.).

Beispiel 9: In analoger Weise wie in Beispiel 1 erhält man ausgehend von 3-Amino-6,7-tetramethylen-4H-1-benzothiopyran-4-on das N-(6,7-Tetramethylen-4H-1-benzothiopyran-4-on-3-yl)-oxamid-methylester vom Smp. 232-233° und
ausgehend von 3-Amino-5,6-tetramethylen-4H-1-benzothiopyran-4-on das N-(5,6-Tetramethylen-4H-1-benzothiopyran-4-on-3-yl)-oxamid-methylester vom Smp. 246-248°.

Das Ausgangsmaterial kann wie folgt hergestellt werden:
Durch Behandeln von 3-(3,4-Tetramethylen-phenylthio)-propionsäure [F.Krollpfeiffer und H.Schultze,Ber. 56, 1822 (1923)] mit konz. Schwefelsäure erhält man ein öliges Isomerengemisch, bestehend aus 6,7-Tetramethylen-2,3-dihydro-4H-1-benzothiopyran-4-on und 5,6-Tetramethylen-2,3-dihydro-4H-1-benzothiopyran-4-on. Dieses Isomerengemisch wird analog Beispiel 1 mit Isopentylnitrit oximiert und anschliessend mit konz. Salzsäure behandelt. Nach Chromatographie des Reaktionsproduktes erhält man 3-Amino-2-chlor-6,7-tetramethylen-4H-1-benzothiopyran-4-on vom Smp. 175-176° und 3-Amino-2-chlor-5,6-tetramethylen-4H-1-benzothiopyran-4-on vom Smp. 135,5-136,5°. Beide Verbindungen werden analog Beispiel 1 zu 3-Amino-6,7-tetramethylen-4H-1-benzothiopyran-4-on, Smp. 147-148°, und 3-Amino-5,6-tetramethylen-4H-1-benzothiopyran-4-on, Smp.113-114°, hydriert.

Beispiel 10: In analoger Weise wie in Beispiel 2 erhält man:
N-(6,7-Tetramethylen-4H-1-benzothiopyran-4-on-3-yl)-oxalsäuremonoamid-hemihydrat vom Smp. 211° (Zers.) und
N-(5,6-Tetramethylen-4H-1-benzothiopyran-4-on-3-yl)-oxalsäuremonoamid vom Smp. 210-211° (Zers.).

Beispiel 11: Zu einer Lösung von 6,4 g (0,02 Mol) N-[6-(n-Butyl)-4H-1-benzothiopyran-4-on-3-yl]-oxamid-methylester in 240 ml Chloroform wird innerhalb einer Stunde eine Lösung von 9,2 g (0,048 Mol) m-Chlorperbenzoesäure (90%ig) zugetropft. Anschliessend wird das Reaktionsgemisch 16 Stunden bei Raumtemperatur weiter gerührt, dann zweimal mit 0,1 N Natriumbicarbonatlösung ausgeschüttelt, mit wasserfreiem Magnesiumsulfat getrocknet und auf etwa 80 ml eingeengt. Diese Lösung wird über eine mit Kieselgel gefüllte Säule filtriert, wobei mit Methylenchlorid eluiert wird. Die einzelnen Fraktionen, je ca. 300 ml, werden dünnschichtchromatographisch kontrolliert. Die Fraktionen 1-7 werden vereinigt, das Lösungsmittel abdestilliert, der

verbleibende Rückstand mit Ether verrührt und abgenutscht. Das so gewonnene Produkt wird aus Essigester umkristallisiert. Man erhält so reines N-[6-(n-Butyl)-4H-1-benzothiopyran-1,1-dioxid-4-on-3-yl]-oxamid-methylester vom Smp. 141,5-142,5°.

Beispiel 12: Zu einer Lösung von 5,05 g (0,0158 Mol) N-[6-(n-Butyl)-4H-1-benzothiopyran-4-on-3-yl]-oxalsäureamid-methylester in Chloroform wird innerhalb von 30 Minuten eine Lösung von 3,03 g (0,0158 Mol) m-Chlorperbenzoesäure (90%ig) zugetropft. Anschliessend wird das Reaktionsgemisch 22 Stunden bei Raumtemperatur gerührt, dann zur Trockene eingedampft, der Rückstand in Methylenchlorid gelöst und die Lösung über eine mit Kieselgel gefüllte Säule filtriert. Es wird mit Methylenchlorid eluiert. Die einzelnen Fraktionen, je etwa 300 ml, werden dünnschichtchromatographisch kontrolliert. Die Fraktionen 9-15 werden vereinigt, das Lösungsmittel abdestilliert und der verbleibende Rückstand mit Ether verrührt. Man erhält so den N-[6-(n-Butyl)-1-oxido-4H-1-benzothiopyran-4-on-3-yl]-oxalsäureamid-methylester vom Smp. 201-202°.

Beispiel 13: 5,25 g (0,02 Mol) N-(6-Methyl-4H-1-benzothiopyran-4-on-3-yl)-oxalsäuremonoamid werden in 40 ml wasserfreiem Dimethylformamid suspendiert und mit 2,66 g (0,022 Mol) Tris-(hydroxymethyl)-aminomethan versetzt und auf 70° erwärmt. Man erhält so eine klare Lösung. Diese wird in 500 ml Aceton gegossen, das auskristallisierte Salz abgenutscht, mit Aceton gewaschen und getrocknet. Man erhält so analysenreines Tris-(hydroxymethyl)-aminomethan-Salz von N-(6-Methyl-4H-1-benzothiopyran-4-on-3-yl)-oxalsäuremonoamid vom Smp. 198° (Zers.).

Beispiel 14: 3,9 g (0,015 Mol) N-(6-Methyl-4H-1-benzothiopyran-4-on-3-yl)-oxalsäuremonoamid werden in 60 ml wasserfreiem Dimethylformamid suspendiert und mit 3,2 g (0,0164 Mol) N-Methyl-D-glucamin versetzt und auf 70° erwärmt. Man erhält so eine klare Lösung. Beim Abkühlen kristallisiert das Salz aus. Man versetzt mit 150 ml Aceton und nutscht das Salz ab. Anschliessend wird es noch aus ca. 160 ml Methanol umkristallisiert. Man erhält so analysenreines N-Methyl-D-glucaminsalz von N-(6-Methyl-4H-1-benzothiopyran-4-on-3-yl)-oxalsäuremonoamid. Es schmilzt bei 126° (Zers.).

Beispiel 15: Nach einer der in der Beschreibung aufgeführten Verfahrensweise kann man neben den in den Beispielen 1-14 genannten neuen Verbindungen ferner herstellen:

N-(2,6-Dimethyl-4H-1-benzothiopyran-4-on-3-yl)-oxalsäureamid-methylester,

N-(2,6-Dimethyl-4H-1-benzothiopyran-4-on-3-yl)-oxalsäuremonoamid,

N-(2-Methyl-4H-1-benzothiopyran-4-on-3-yl)-oxalsäuremonoamid, Smp. 195°,

N-(2-Methyl-6,7-trimethylen-4H-1-benzothiopyran-4-on-3-yl)-oxalsäuremonoamid,

N-(6-Fluor-4H-1-benzothiopyran-4-on-3-yl)-oxalsäuremonoamid, Smp. 235° (Zers.), z.B. ausgehend von 3-Amino-6-fluor-4H-1-benzothiopyran-4-on, Smp. 168-169°,

N-(6-Fluoro-4H-1-benzothiopyran-4-on-3-yl)-oxalsäuremethylester, Smp. 294° (Zers.),

N-(6-Methoxy-4H-1-benzothiopyran-4-on-3-yl)-oxalsäuremethylester, Smp. 264-266°,

N-(6-Methoxy-4H-1-benzothiopyran-4-on-3-yl)-oxalsäuremonoamid, Smp. 234° (Zers.),

N-(2-Methyl-4H-1-benzothiopyran-4-on-3-yl)-oxalsäureamid-methylester, Smp. 205-207° (Zers.).

Beispiel 16: 1,53 g (0,005 Mol) N-[6-(n-Butyl)-4H-1-benzothiopyran-4-on-3-yl]-2-hydroxy-essigsäuremonoamid werden in 60 ml Aceton gelöst und mit 1 g Kaliumpermanganat in 50 ml Wasser bei Raumtemperatur 48 Stunden gerührt. Das Reaktionsgemisch wird unter vermindertem Druck zur Trockene eingedampft, der Rückstand in Methylenchlorid aufgenommen und mit Wasser gewaschen. Nach Trocknen der organischen Phase über Natriumsulfat wird eingeengt. Man erhält so das N-[6-(n-Butyl)-4H-1-benzothiopyran-4-on-3-yl]-oxalsäuremonoamid vom Smp. 208° (Zers.).

Beispiel 17: 0,3 g (0,0001 Mol) N-[6-(n-Butyl)-4H-1-benzothiopyran-4-on-3-yl]-oxalsäuremonoamid wird in 20 ml mit Salzsäuregas gesättigtes wasserfreies Methanol gelöst und 12 Stunden unter Rückfluss erhitzt, abgekühlt und unter vermindertem Druck zur Trockene eingedampft. Den Rückstand versetzt man mit 10 ml Wasser und stellt mit wässriger konzentrierter Ammoniaklösung alkalisch. Man extrahiert zweimal mit 20 ml Ethylacetat und wäscht die organische Phase bei 5° mit 10 ml 2N Kaliumbicarbonat und 10 ml Wasser, trocknet über Magnesiumsulfat und engt unter vermindertem Druck zur Trockene ein. Man erhält so N-[6-(n-Butyl)-4H-1-benzothiopyran-4-on-3-yl]-oxalsäureamid-methylester vom Smp. 192-193°.

Beispiel 18: 1 g N-[6-(n-Butyl)-4H-1-benzothiopyran-4-on-3-yl]-trichloracetamid werden in 100 ml Ethanol und 10 ml 25%ige Natronlauge 5 Stunden unter Rückfluss erhitzt. Das Lösungsmittel wird im Vakuum abgezogen und der wässrige Rückstand mit 2 N Salzsäure angesäuert. Man erhält so N-[6-(n-Butyl)-4H-1-benzothiopyran-4-on-3-yl]-oxalsäuremonoamid vom Smp. 208° (Zers.).

Das Ausgangsmaterial kann in analoger Weise wie im Beispiel 1 beschrieben durch Umsetzung von 3-Amino-6-(n-butyl)-4H-1-benzothiopyran-4-on und Trichloracetylchlorid hergestellt werden.

Beispiel 19: 1,2 g (0,005 Mol) N-(4H-1-Benzothiopyran-4-on-3-yl)-2-methoxy-essigsäuremonoamid werden in 60 ml Aceton gelöst und mit 25 ml Wasser versetzt. Dann wird bei Raumtemperatur unter kräftigem Rühren portionenweise Kaliumpermanganat zugesetzt bis keine Entfärbung mehr zu beobachten ist. Anschliessend wird 12 Stunden weiter gerührt und filtriert. Das Filtrat wird bei vermindertem Druck und 50° zur Trockene eingeengt. Der Rückstand wird mit 20 ml Eiswasser versetzt und mit Chloroform aufgenommen. Die organische Phase wird gewaschen, über Natriumfulfat getrocknet und unter vermindertem Druck zur Trockene eingeengt. Man erhält so den N-(4H-1-Benzothiopyran-4-on-3-yl)-oxalsäureamid-methylester vom Smp. 192-193°.

Das Ausgangsmaterial kann in analoger Weise wie in Beispiel 1 beschrieben, durch Umsetzung von 3-Amino-4H-1-benzothiopyran-4-on und 2-Methoxyessigsäure hergestellt werden.

Beispiel 20: 6,1 g (0,025 Mol) N-[6-Methyl-4H-1-benzothiopyran-4-on-3-yl]-cyanoformamid werden in 75 ml 90°iger Schwefelsäure gelöst und die Lösung über Nacht bei Raumtemperatur gerührt. Anschliessend giesst man das Reakionsgemisch auf Eis und nutscht das ausgefallene Produkt ab. Man wäscht es zuerst mit Wasser, dann mit Ethanol. Nach dem Trocknen erhält man N-[6-Methyl-4H-1-benzothiopyran-4-on-3-yl]-oxalsäuremonoamid vom Smp. 222° (Zers.).

Das N-[6-Methyl-4H-1-benzothiopyran-4-on-3-yl]-cyanoformamid kann beispielsweise aus 3-Amino-6-methyl-4H-1-benzothiopyran-4-on und Cyanformylchlorid [R.Appel et al., Angew.Chem. 95 (1983)807] hergestellt werden.

Beispiel 21: 2,4 g (0,01 Mol) N-[6-Methyl-4H-1-benzothiopyran-4-on-3-yl]-cyanoformamid wird in 50 ml Methanol suspendiert und 14 Stunden bei 80° erhitzt (Glasbombenrohr). Anschliessend wird das Reaktionsgemisch weitgehend eingeengt, abgenutscht, zuerst mit wenig Methanol, dann mit Ether gewaschen und getrocknet. Man erhält so den N-(6-Methyl-4H-1-benzothiopyran-4-on-3-yl)-oxalsäureamid-methylester vom Smp. 238-240°C.

Beispiel 22: 9,6 g (0,05 Mol) 3-Amino-6-methyl-4H-1-benzothiopyran-4-on, 0,52 g (0,005 Mol) Oxalsäuremonomethylester und 11,3 g (0,055 Mol) N,N'-Dicyclohexylcarbodiimid werden in 150 ml Tetrahydrofuran gelöst und 6 Stunden bei Raumtemperatur gerührt. Danach dampft man das Reaktionsgemisch zur Trockene, verrührt den Rückstand mit 250 ml Methylenchlorid, nutscht vom ungelösten N,N'-Dicyclohexylharnstoff ab, engt das Filtrat ein, und chromatographiert über eine mit Kieselgel gefüllte Säule. Man eluiert mit Methylenchlorid und erhält so N-(6-Methyl-4H-1-benzothiopyran-4-on-3-yl)-oxalsäureamid-methylester, vom Smp. 237-239°.

Beispiel 23: 5,5 g (0,02 Mol) N-(4H-2,3-Dihydro-1-benzothiopyran-4-on-3-yl)-oxalsäureamid-methylester werden in ein Gemisch von 80 ml Eisessig und 10 ml Essigsäureanhydrid gelöst. Anschliessend werden bei ca. 100° 6,9 g (0,02 Mol) Tritylperchlorat zugegeben. Man hält das Reaktionsgemisch noch im ganzen etwa 30 Min. bei 100°, dampft im Wasserstrahlvakuum zur Trockene ein und zieht mit Ether aus. Der Extraktionsrückstand wird mit 150 ml gesättigter Natriumbikarbonatlösung verrieben, abgenutscht, mit Wasser gewaschen und getrocknet. Anschliessend wird das Rohprodukt aus 2-Ethoxyethanol umkristallisiert. Man erhält so N-(4H-1-Benzothiopyran-4-on-3-yl)-oxalsäureamid-methylester vom Smp. 258°.

Das Ausgangsmaterial kann wie folgt hergestellt werden:
Zu einer Suspension von 3,7 g (0,017 Mol) 3-Amino-2,3-dihydro-4H-1-benzothiopyran-4-on-hydrochlorid in 250 ml Chloroform gibt man 2,3 g (0,019 Mol) Oxalsäuremonomethylesterchlorid und tropft anschliessend noch 3,8 g Triethylamin zu. Man rührt noch eine Stunde bei Raumtemperatur, schüttelt dann das Reaktionsgemisch zwei mal mit je 100 ml Wasser, trocknet die Chloroformphase mit wasserfreiem Magnesiumsulfat und dampft zur Trockene ein. Das zurückgebliebene Oel wird in Methylenchlorid gelöst und über eine mit Kieselgel gefüllte Säule chromatographiert. Man erhält so N-(4H-2,3-Dihydro-1-benzothiopyran-4-on-3-yl)-oxalsäureamid-methylester vom Smp. 107-108°.

Beispiel 24: Eine Lösung von 8,9 g (0,025 Mol) N-[2-Chlor-6-(n-butyl)-4H-1-benzothiopyran-4-on-3-yl]-oxalsäureamid-methylester und 2,8 g Triethylamin in 200 ml Ethanol wird bei 20-25° in Gegenwart von 1 g 10%iger Palladiumkohle bei Normaldruck hydriert. Der Katalysator wird abfiltriert, das Filtrat zur Trockene eingedampft und der Rückstand aus 2-Ethoxyethanol umkristallisiert. Man erhält so N-[6-(n-Butyl)-4H-1-benzothiopyran-4-on-3-yl]-oxalsäureamid-methylester vom Smp. 193-195°.

Das Ausgangsmaterial N-[2-Chlor-6-(n-butyl)-4H-1-benzothiopyran-4-on-3-yl]-oxalsäureamid-methylester kann analog wie im Beispiel 1 beschrieben, durch Umsetzung von 6,7 g (0,025 Mol) 2-Chlor-3-amino-6-(n-butyl)-4H-1-benzothiopyran-4-on mit 3,4 g (0,028 Mol) Oxalsäuremonomethylesterchlorid in Gegenwart von 2,8 g (0,028 Mol) Triethylamin gewonnen werden.

Beispiel 25: Eine zur Inhalation geeignete, 2%ige wässrige Lösung von N-(6-Methyl-4H-1-benzothiopyran-4-on-3-yl)-oxalsäuremonoamid kann wie

folgt hergestellt werden. (Zusammensetzung für 100 ml)

| | | |
|---|---|---|
| Wirkstoff | | 2,000 g |
| Dinatriumsalz der Ethylendiamintetra-essigsäure (Stabilisator) | | 0,010 g |
| Benzalkoniumchlorid (Konservierungs-mittel) | | 0,010 g |
| Wasser, destilliert | ad | 100 ml |

Der Wirkstoff wird in frisch destilliertem Wasser gelöst und die Lösung mit dem Dinatriumsalz der Ethylendiamintetraessigsäure und dem Benzalkoniumchlorid, das ist ein Gemisch von Alkyl-methyl-benzylammoniumchloriden, worin Alkyl von 8-18 Kohlenstoffatome enthält, versetzt. Nach vollständiger Auflösung der Komponenten wird die erhaltene Lösung mit Wasser auf ein Volumen von 100 ml gebracht, abgefüllt und gasdicht verschlossen.

Beispiel 26: Zur Insufflation geeignete, 0,025 g N-(6-Methyl-4H-1-benzothiopyran-4-on-3-yl)-oxalsäuremonoamid enthaltende Kapseln, können wie folgt hergestellt werden: (Zusammensetzung für 1000 Kapseln)

| | |
|---|---|
| Wirkstoff | 25,00 g |
| Laktose, gemahlen | 25,00 g |

Der Wirkstoff und die Laktose (feinst gemahlen) werden gut miteinander vermischt. Das erhaltene Pulver wird gesiebt und in Portionen zu je 0,05 g in Gelatinekapseln abgefüllt.

Beispiel 27: Tabletten enthaltend 100 mg N-(6,7-Trimethylen-4H-1-benzothiopyran-4-on-3-yl)-oxalsäuremonoamid als Wirkstoff können beispielsweise in folgender Zusammensetzung hergestellt werden:

| Zusammensetzung | pro Tablette |
|---|---|
| Wirkstoff | 100 mg |
| Milchzucker | 50 mg |
| Weizenstärke | 73 mg |
| Kolloidale Kieselsäure | 13 mg |
| Talk | 12 mg |
| Magnesiumstearat | 2 mg |
| | 250 mg |

Herstellung

Der Wirkstoff wird mit dem Milchzucker, einem Teil der Weizenstärke und mit kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist. Diese Masse wird durch ein Sieb von etwa 1 mm Maschenweite getrieben, getrocknet, und das trockene Granulat nochmals durch ein Sieb getrieben. Dann werden die restliche Weizenstärke, der Talk und das Magnesiumstearat zugemischt. Die erhaltene Tablettiermischung wird zu Tabletten von je 250 mg mit Bruchkerbe(n) verpresst.

Beispiel 28: In analoger Weise wie in den Beispielen 25 bis 27 beschrieben, können auch pharmazeutische Präparate enthaltend eine andere Verbindung gemäss einem der Beispiele 1-24 hergestellt werden.

Patentansprüche für die Vertragsstaaten: BE, CH, LI, DE, FR, GB, IT, LU, NL, SE

1. Neue Benzothiopyranone der allgemeinen Formel

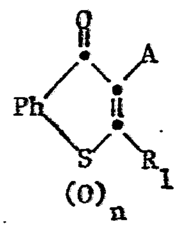

(I),

worin $R_1$ Wasserstoff oder einen aliphatischen Rest bedeutet, n für 0, 1 oder 2 steht, Ph substituiertes oder unsubstituiertes 1,2-Phenylen darstellt und A eine Gruppe der Formel -NH-CO-R bedeutet, in der R Carboxy oder verestertes Carboxy darstellt, und ihre Salze.

2. Verbindungen gemäss Patentanspruch 1 der Formel I, worin $R_1$ Wasserstoff, Niederalkyl oder Niederalkenyl bedeutet, n für 0, 1 oder 2 steht, Ph ein- oder mehrfach durch Niederalkyl, Niederalkenyl, Niederalkinyl, zwei benachbarte C-Atome überbrückendes 3- oder 4-gliedriges Niederalkylen, Hydroxyniederalkyl, Halogenniederalkyl, Hydroxy, Niederalkoxy, Hydroxyniederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes oder unsubstituiertes 1,2-Phenylen darstellt und A eine Gruppe der Formel -NH-CO-R bedeutet, in der R Carboxy, Niederalkoxycarbonyl, unsubstituiertes oder ein- oder mehrfach durch Niederalkyl, Niederalkenyl, Niederalkinyl, zwei benachbarte C-Atome überbrückendes 3- oder 4-gliedriges Niederalkylen, Hydroxyniederalkyl, Halogenniederalkyl, Hydroxy, Niederalkoxy, Hydroxyniederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes Phenoxycarbonyl darstellt, und Salze.

3. Verbindungen gemäss Patentanspruch 1 der Formel I, worin $R_1$ Wasserstoff oder Niederalkyl bedeutet, n für 0, 1 oder 2 steht, Ph ein- oder mehrfach durch Niederalkyl, Niederalkenyl, zwei benachbarte

C-Atome überbrückendes 3- oder 4-gliedriges Niederalkylen, Hydroxyniederalkyl, Hydroxy, Niederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes oder unsubstituiertes 1,2-Phenylen darstellt und A eine Gruppe der Formel -NH-CO-R bedeutet, in der R Carboxy oder Niederalkoxycarbonyl darstellt, und Salze.

4. Verbindungen gemäss Patentanspruch 1 der Formel I, worin $R_1$ Wasserstoff oder Niederalkyl bedeutet, n für 0 steht, Ph ein- oder mehrfach durch Niederalkyl, Niederalkenyl, zwei benachbarte C-Atome überbrückendes 3- oder 4-gliedriges Niederalkylen, Niederalkoxy und/oder Halogen substituiertes oder unsubstituiertes 1,2-Phenylen darstellt und A eine Gruppe der Formel -NH-CO-R bedeutet, in der R Carboxy oder Niederalkoxycarbonyl darstellt, und Salze.

5. Verbindungen gemäss Patentanspruch 1 der Formel I, worin $R_1$ Wasserstoff bedeutet, n für 0 steht, Ph ein- oder mehrfach durch Niederalkyl, zwei benachbarte C-Atome überbrückendes 3- oder 4-gliedriges Niederalkylen mit 3 bis und mit 7 C-Atomen, Niederalkoxy und/oder Halogen substituiertes oder unsubstituiertes 1,2-Phenylen darstellt und A eine Gruppe der Formel -NH-CO-R bedeutet, in der R Carboxy oder Niederalkoxycarbonyl darstellt, und Salze.

6. Verbindungen gemäss Patentanspruch 1 der Formel

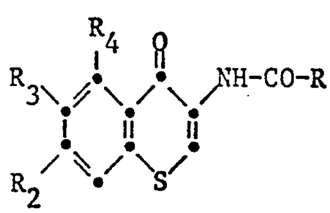

(Ia),

worin R Carboxy bedeutet, $R_2$ und $R_4$ Wasserstoff darstellen und $R_3$ Wasserstoff, Niederalkyl mit bis und mit 4 C-Atomen oder Niederalkoxy mit bis und mit 4 C-Atomen bedeutet oder

worin R Carboxy darstellt, $R_2$ und $R_3$ gemeinsam Tri- oder Tetramethylen darstellen und $R_4$ Wasserstoff bedeutet, und Salze.

7. Verbindungen gemäss Patentanspruch 1 der Formel (Ia), worin R Carboxy bedeutet, $R_2$ und $R_4$ Wasserstoff bedeuten und $R_3$ Niederalkyl mit bis und mit 4 C-Atomen bedeutet oder worin $R_2$ und $R_3$ gemeinsam Tri- oder Tetramethylen bedeuten und $R_4$ Wasserstoff ist oder worin $R_3$ und $R_4$ gemeinsam Tri- oder Tetramethylen bedeuten und $R_2$ Wasserstoff ist, und ihre Salze.

8. Verbindungen gemäss Patentanspruch 1 der Formel (Ia), worin R Carboxy bedeutet, $R_2$ und $R_4$ Wasserstoff bedeuten und $R_3$ Niederalkyl mit bis und mit 4 C-Atomen, insbesondere Methyl, bedeutet oder worin $R_2$ und $R_3$ gemeinsam Tri- oder Tetramethylen bedeuten und $R_4$ Wasserstoff ist, und ihre Salze.

9. N-[6-(n-Butyl)-4H-1-benzothiopyran-4-on-3-yl]-oxalsäureamid-methylester, N-[6-(n-Butyl)-4H-1-benzothiopyran-4-on-3-yl]-oxalsäuremonoamid oder ein Salz davon, N-(4H-1-Benzothiopyran-4-on-3-yl)-oxamid-methylester, N-(6-Chlor-4H-1-benzothiopyran-4-on-3-yl)-oxamid-methylester, N-(6-Methyl-4H-1-benzothiopyran-4-on-3-yl)-oxamid-methylester, N-(6,7-Trimethylen-4H-1-benzothiopyran-4-on-3-yl)-oxamid-methylester, N-(5,6-Trimethylen-4H-1-benzothiopyran-4-on-3-yl)-oxamid-methylester, N-(5,7-Dimethyl-4H-1-benzothiopyran-4-on-3-yl)-oxamid-methylester, N-(6-Ethyl-4H-1-benzothiopyran-4-on-3-yl)-oxamid-methylester, N-(6-Chlor-4H-1-benzothiopyran-4-on-3-yl)-oxalsäuremonoamid oder ein Salz davon, N-(6,7-Tetramethylen-4H-1-benzothiopyran-4-on-3-yl)-oxamid-methylester, N-(5,6-Tetramethylen-4H-1-benzothiopyran-4-on-3-yl)-oxamid-methylester, N-[6-(n-Butyl)-4H-1-benzothiopyran-1,1-dioxid-4-on-3-yl]-oxamid-methylester, N-[6-(n-Butyl)-1-oxido-4H-1-benzothiopyran-4-on-3-yl]-oxalsäureamid-methylester, N-(2,6-Dimethyl-4H-1-benzothiopyran-4-on-3-yl)-oxalsäureamid-methylester, N-(2,6-Dimethyl-4H-1-benzothio-

pyran-4-on-3-yl)-oxalsäuremonoamid oder ein Salz davon, N-(2-Methyl-4H-1-benzothiopyran-4-on-3-yl)-oxalsäuremonoamid oder ein Salz davon, N-(2-Methyl-6,7-trimethylen-4H-1-benzothiopyran-4-on-3-yl)-oxalsäuremonoamid oder ein Salz davon, N-(6-Fluor-4H-1-benzothiopyran-4-on-3-yl)-oxalsäuremonoamid oder ein Salz davon, N-(6-Fluor-4H-1-benzothiopyran-4-on-3-yl)-oxalsäuremethylester, N-(6-Methoxy-4H-1-benzothiopyran-4-on-3-yl)-oxalsäuremethylester oder N-(6-Methoxy-4H-1-benzothiopyran-4-on-3-yl)-oxalsäuremonoamid oder ein Salz davon.

10. N-(4H-1-Benzothiopyran-4-on-3-yl)-oxalsäuremonoamid oder ein Salz davon.

11. N-(6-Methyl-4H-1-benzothiopyran-4-on-3-yl)-oxalsäuremonoamid oder ein Salz davon.

12. N-(6,7-Trimethylen-4H-1-benzothiopyran-4-on-3-yl)-oxalsäuremonoamid oder ein Salz davon.

13. N-(5,6-Trimethylen-4H-1-benzothiopyran-4-on-3-yl)-oxalsäuremonoamid oder ein Salz davon.

14. N-(5,7-Dimethyl-4H-1-benzothiopyran-4-on-3-yl)-oxalsäuremonoamid oder ein Salz davon.

15. N-(6-Ethyl-4H-1-benzothiopyran-4-on-3-yl)-oxalsäuremonoamid oder ein Salz davon.

16. N-(6,7-Tetramethylen-4H-1-benzothiopyran-4-on-3-yl)-oxalsäuremonoamid oder ein Salz davon.

17. N-(5,6-Tetramethylen-4H-1-benzothiopyran-4-on-3-yl)-oxalsäuremonoamid oder ein Salz davon.

18. Verbindung gemäss einem der Patentansprüche 1-17 oder ein pharmazeutisch verwendbares Salz davon zur Behandlung von Allergien.

19. Verbindung gemäss einem der Patentansprüche 1-18 oder ein pharmazeutisch verwendbares Salz davon zur Anwendung in einem Verfahren therapeutischen und prophylaktischen Behandlung des menschlichen oder tierischen Körpers.

20. Pharmazeutische Präparate, enthaltend mindestens eine Verbindung gemäss einem der Patentansprüche 1-19 oder ein pharmazeutisch verwendbares Salz davon.

21. Verwendung von Verbindungen gemäss einem der Patentansprüche 1-19 zur Herstellung pharmazeutischer Präparate.

22. Verfahren zur Herstellung von Verbindungen der Formel (I) gemäss Anspruch 1, dadurch gekennzeichnet, dass man in einer Verbindung der Formel

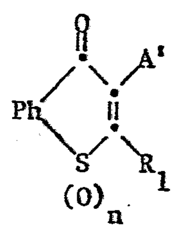

(II),

worin n, Ph und $R_1$ die angegebenen Bedeutungen haben und A' eine in A überführbare Gruppe bedeutet, A' in A überführt oder

eine Verbindung der Formel

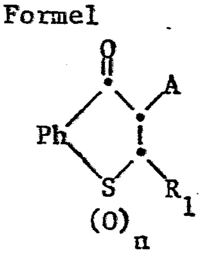

(IV),

worin n, Ph, A und $R_1$ die angegebenen Bedeutungen haben, oder ein Salz davon zu der entsprechenden Verbindung der Formel (I) dehydriert oder in einer Verbindung der Formel

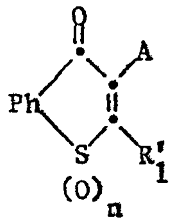

(V),

worin n, Ph und A die angegebenen Bedeutungen haben, und $R_1'$ einen in $R_1$ überführbaren Rest bedeutet, den Rest $R_1'$ in $R_1$ überführt oder

eine Verbindung der Formel

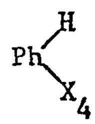

(VI),

worin Ph die angegebene Bedeutung hat und $X_4$ eine Gruppe der Formel $-S(O)_n-C(R_1)=C(A)-X_5$ bedeutet, in der $X_5$ gegebenenfalls funktionell abgewandeltes Carboxy bedeutet, oder ein Salz davon cyclisiert oder in einer Verbindung der Formel

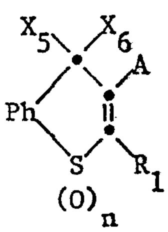

(VII),

worin A, $R_1$, n und Ph die angegebenen Bedeutungen haben und $X_5$ und $X_6$ gemeinsam in die Oxogruppe überführbare Reste darstellen, oder einem Salz davon $X_5$ und $X_6$ in die Oxogruppe überführt und, wenn erwünscht, eine verfahrensgemäss erhältliche freie Verbindung in eine andere freie Verbindung überführt, ein verfahrensgemäss erhältliches Salz in die freie Verbindung oder in ein anderes Salz überführt, eine verfahrensgemäss erhältliche freie Verbindung in ein Salz überführt und gewünschtenfalls ein verfahrensgemäss erhältliches Isomerengemisch in seine Komponenten auftrennt.

23. Verfahren gemäss Anspruch 22, dadurch gekennzeichnet, dass man von einer auf irgendeiner Stufe des Verfahrens als Zwischenstufe erhältlichen Verbindung ausgeht und die fehlenden Schritte durchführt und/oder einen Ausgangsstoff in Form eines Salzes, Isomeren und/oder Racemates bzw. Antipoden verwendet oder insbesondere unter den Reaktionsbedingungen bildet.

24. Verfahren zur Herstellung pharmazeutischer Präparate, dadurch gekennzeichnet, dass man eine Verbindung gemäss einem der Ansprüche 1-19 mit üblichen Hilfs- und Trägerstoffen vermischt.

25. Die nach dem Verfahren gemäss Anspruch 22 erhältlichen Verbindungen.

Patentansprüche für den Vertragsstaat: AT

1. Verfahren zur Herstellung von Verbindungen der Formel

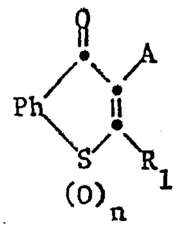

(I),

worin $R_1$ Wasserstoff oder einen aliphatischen Rest bedeutet, n für 0, 1 oder 2 steht, Ph substituiertes oder unsubstituiertes 1,2-Phenylen darstellt und A eine Gruppe der Formel -NH-CO-R bedeutet, in der R Carboxy oder verestertes Carboxy darstellt, und ihrer Salze, dadurch gekennzeichnet, dass man in einer Verbindung der Formel

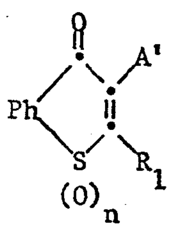

(II),

worin n, Ph und $R_1$ die angegebenen Bedeutungen haben und A' eine in A überführbare Gruppe bedeutet, A' in A überführt oder

eine Verbindung der Formel

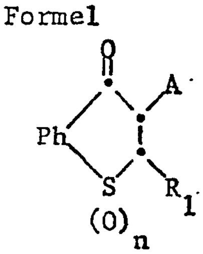

(IV),

worin n, Ph, A und $R_1$ die angegebenen Bedeutungen haben, oder ein Salz davon zu der entsprechenden Verbindung der Formel (I) dehydriert oder in einer Verbindung der Formel

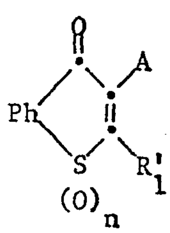

(V),

worin n, Ph und A die angegebenen Bedeutungen haben, und $R_1'$ einen in $R_1$ überführbaren Rest bedeutet, den Rest $R_1'$ in $R_1$ überführt oder

eine Verbindung der Formel

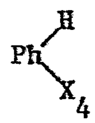

(VI),

worin Ph die angegebene Bedeutung hat und $X_4$ eine Gruppe der Formel $-S(O)_n-C(R_1)=C(A)-X_5$ bedeutet, in der $X_5$ gegebenenfalls funktionell abgewandeltes Carboxy bedeutet, oder ein Salz davon cyclisiert oder in einer Verbindung der Formel

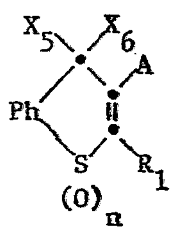

(VII),

worin A, $R_1$, n und Ph die angegebenen Bedeutungen haben und $X_5$ und $X_6$ gemeinsam in die Oxogruppe überführbare Reste darstellen, oder einem Salz davon $X_5$ und $X_6$ in die Oxogruppe überführt und, wenn erwünscht, eine verfahrensgemäss erhältliche freie Verbindung in eine andere freie Verbindung überführt, ein verfahrensgemäss erhältliches Salz in die freie Verbindung oder in ein anderes Salz überführt, eine verfahrensgemäss erhältliche freie Verbindung in ein Salz überführt und gewünschtenfalls ein verfahrensgemäss erhältliches Isomerengemisch in seine Komponenten auftrennt.

2. Verfahren gemäss Anspruch 1, dadurch gekennzeichnet, dass man von einer auf irgendeiner Stufe des Verfahrens als Zwischenstufe erhältlichen Verbindung ausgeht und die fehlenden Schritte durchführt und/oder einen Ausgangsstoff in Form eines Salzes, Isomeren und/oder Racemates bzw. Antipoden verwendet oder insbesondere unter den Reaktionsbedingungen bildet.

3. Verfahren nach Anspruch 1 oder 2 zur Herstellung von Verbindungen der Formel I, worin $R_1$ Wasserstoff, Niederalkyl oder Niederalkenyl bedeutet, n für 0, 1 oder 2 steht, Ph ein- oder mehrfach durch Niederalkyl, Niederalkenyl, Niederalkinyl, zwei benachbarte C-Atome überbrückendes 3- oder 4-gliedriges Niederalkylen, Hydroxyniederalkyl, Halogenniederalkyl, Hydroxy, Niederalkoxy, Hydroxyniederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes oder unsubstituiertes 1,2-Phenylen darstellt und A eine Gruppe der Formel -NH-CO-R bedeutet, in der R Carboxy, Niederalkoxycarbonyl, unsubstituiertes oder ein- oder mehrfach durch Niederalkyl, Niederalkenyl, Niederalkinyl, zwei benachbarte C-Atome überbrückendes 3- oder 4-gliedriges Niederalkylen, Hydroxyniederalkyl, Halogenniederalkyl, Hydroxy, Niederalkoxy, Hydroxyniederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes Phenoxycarbonyl darstellt, und ihrer Salze.

4. Verfahren nach Anspruch 1 oder 2 zur Herstellung von Verbindungen der Formel I, worin $R_1$ Wasserstoff oder Niederalkyl bedeutet, n für 0, 1 oder 2 steht, Ph ein- oder mehrfach durch Niederalkyl, Niederalkenyl, zwei benachbarte C-Atome überbrückendes 3- oder 4-gliedriges Niederalkylen, Hydroxyniederalkyl, Hydroxy, Niederalkoxy, Niederalkenyloxy, Niederalkylthio, Niederalkansulfinyl, Niederalkansulfonyl, Niederalkanoyl, Niederalkanoyloxy, Nitro und/oder Halogen substituiertes oder unsubstituiertes 1,2-Phenylen darstellt und A eine Gruppe der Formel -NH-CO-R bedeutet, in der R Carboxy oder Niederalkoxycarbonyl darstellt, und ihrer Salze.

5. Verfahren nach Anspruch 1 oder 2 zur Herstellung von Verbindungen der Formel I, worin $R_1$ Wasserstoff oder Niederalkyl bedeutet, n für 0 steht, Ph ein- oder mehrfach durch Niederalkyl, Niederalkenyl, zwei benachbarte C-Atome überbrückendes 3- oder 4-gliedriges Niederalkylen,

Niederalkoxy und/oder Halogen substituiertes oder unsubstituiertes 1,2-Phenylen darstellt und A eine Gruppe der Formel -NH-CO-R bedeutet, in der R Carboxy oder Niederalkoxycarbonyl darstellt, und ihrer Salze.

6. Verfahren nach Anspruch 1 oder 2 zur Herstellung von Verbindungen der Formel I, worin $R_1$ Wasserstoff bedeutet, n für 0 steht, Ph ein- oder mehrfach durch Niederalkyl, zwei benachbarte C-Atome überbrückendes 3- oder 4-gliedriges Niederalkylen mit 3 bis und mit 7 C-Atomen, Niederalkoxy und/oder Halogen substituiertes oder unsubstituiertes 1,2-Phenylen darstellt und A eine Gruppe der Formel -NH-CO-R bedeutet, in der R Carboxy oder Niederalkoxycarbonyl darstellt, und ihrer Salze.

7. Verfahren nach Anspruch 1 oder 2 zur Herstellung von Verbindungen der Formel Ia

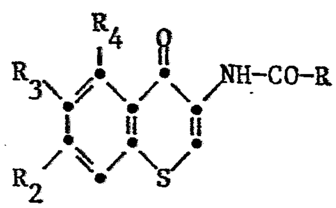

(Ia),

worin R Carboxy bedeutet, $R_2$ und $R_4$ Wasserstoff darstellen und $R_3$ Wasserstoff, Niederalkyl mit bis und mit 4 C-Atomen oder Niederalkoxy mit bis und mit 4 C-Atomen bedeutet oder worin R Carboxy darstellt, $R_2$ und $R_3$ gemeinsam Tri- oder Tetramethylen darstellen und $R_4$ Wasserstoff bedeutet, und ihrer Salze.

8. Verfahren nach Anspruch 1 oder 2 zur Herstellung von Verbindungen der Formel (Ia), worin R Carboxy bedeutet, $R_2$ und $R_4$ Wasserstoff bedeuten und $R_3$ Niederalkyl mit bis und mit 4 C-Atomen bedeutet oder worin $R_2$ und $R_3$ gemeinsam Tri- oder Tetramethylen bedeuten und $R_4$ Wasserstoff ist oder worin $R_3$ und $R_4$ gemeinsam Tri- oder Tetramethylen bedeuten und $R_2$ Wasserstoff ist, und ihrer Salze.

9. Verfahren nach Anspruch 1 oder 2 zur Herstellung von Verbindungen der Formel (Ia), worin R Carboxy bedeutet, $R_2$ und $R_4$ Wasserstoff bedeuten und $R_3$ Niederalkyl mit bis und mit 4 C-Atomen, insbesondere Methyl, bedeutet oder worin $R_2$ und $R_3$ gemeinsam Tri- oder Tetramethylen bedeuten und $R_4$ Wasserstoff ist, und ihrer Salze.

10. Verfahren nach Anspruch 1 oder 2 zur Herstellung von N-[6-(n-Butyl)-4H-1-benzothiopyran-4-on-3-yl]-oxalsäureamidmethylester, N-[6-(n-Butyl)-4H-1-Benzothiopyran-4-on-3-yl]-oxalsäuremonoamid oder einem Salz davon, N-(4H-1-benzothiopyran-4-on-3-yl)-oxamid-methylester, N-(6-Chlor-4H-1-benzothiopyran-4-on-3-yl)-oxamid-methylester, N-(6-Methyl-4H-1-benzothiopyran-4-on-yl)-oxamid-methylester, N-(6,7-Trimethylen-4H-1-benzothiopyran-4-on-3-yl)-oxamid-methylester, N-(5,6-Trimethylen-4H-1-benzothiopyran-4-on-3-yl)-oxamid-methylester, N-(5,7-Dimethyl-4H-1-benzothiopyran-4-on-3-yl)-oxamid-methylester, N-(6-Ethyl-4H-1-benzothiopyran-4-on-3-yl)-oxamid-methylester, N-(6-Chlor-4H-1-benzothiopyran-4-on-3-yl)-oxalsäuremonoamid oder ein Salz davon, N-(6,7, Tetramethylen-4H-1-benzothiopyran-4-on-3-yl)-oxamidmethylester, N-(5,6-Tetramethylen-4H-1-benzothiopyran-4-on-3-yl)-oxamidmethylester, N-[6-(n-Butyl)-4H-1-benzothiopyran-1,1-dioxid-4-on-3-yl]-oxamid-methylester, N-[6-(n-Butyl)-1-oxido-4H-1-benzothiopyran-4-on-3-yl]-oxalsäureamid-methylester, N-(2,6-Dimethyl-4H-1-benzothiopyran-4-on-3-yl)-oxalsäureamid-methylester, N-(2,6-Dimethyl-4H-1-benzothiopyran-4-on-3-yl)-oxalsäuremonoamid oder ein Salz davon, N-(2-Methyl-4H-1-benzothiopyran-4-on-3-yl)-oxalsäuremonoamid oder ein Salz davon, N-(2-Methyl-6,7-trimethylen-4H-1-benzothiopyran-4-on-3-yl)-oxalsäuremonoamid oder ein Salz davon, N-(6-Fluor-4H-1-benzothiopyran-4-on-3-yl)-oxalsäuremonoamid oder ein Salz davon, N-(6-Fluor-4H-1-benzothiopyran-4-on-3-yl)-oxalsäuremethylester, N-(6-Methoxy-4H-1-benzothiopyran-4-on-3-yl)-oxalsäuremethylester oder N-(6-Methoxy-4H-1-benzothiopyran-4-on-3-yl)-oxalsäuremonoamid oder ein Salz davon.

11. Verfahren nach Anspruch 1 oder 2 zur Herstellung von N-(4H-1-Benzothiopyran-4-on-3-yl)-oxalsäuremonoamid oder ein Salz davon.

12. Verfahren nach Anspruch 1 oder 2 zur Herstellung von N-(6-Methyl-4H-1-benzothiopyran-4-on-3-yl)-oxalsäuremonoamid oder einem Salz davon.

13. Verfahren nach Anspruch 1 oder 2 zur Herstellung von N-(6,7-Trimethylen-4H-1-benzothiopyran-4-on-3-yl)-oxalsäuremonoamid oder einem Salz davon.

14. Verfahren nach Anspruch 1 oder 2 zur Herstellung von N-(5,6-Trimethylen-4H-1-benzothiopyran-4-on-3-yl)-oxalsäuremonoamid oder einem Salze davon.

15. Verfahren nach Anspruch 1 oder 2 zur Herstellung von N-(5,7-Dimethyl-4H-1-benzothiopyran-4-on-3-yl)-oxalsäuremonoamid oder ein Salz davon.

16. Verfahren nach Anspruch 1 oder 2 zur Herstellung von N-(6-Ethyl-4H-1-benzothiopyran-4-on-3-yl)-oxalsäuremonoamid oder einem Salz davon.

17. Verfahren nach Anspruch 1 oder 2 zur Herstellung von N-(6,7-Tetramethylen-4H-1-benzothiopyran-4-on-3-yl)-oxalsäuremonoamid oder einem Salz davon.

18. Verfahren nach Anspruch 1 oder 2 zur Herstellung von N-(5,6-Tetramethylen-4H-1-benzothiopyran-4-on-3-yl)-oxalsäuremonoamid oder einem Salz davon.

19. Verfahren zur Herstellung pharmazeutischer Präparate, dadurch gekennzeichnet, dass man eine Verbindung gemäss einem der Ansprüche 1-18 mit üblichen Hilfs- und Trägerstoffen vermischt.

20. Die nach dem Verfahren gemäss Anspruch 1 erhältlichen Verbindungen.